# EUROPÄISCHE PATENTSCHRIFT

(11) **EP 1 318 193 B1**
(45) Veröffentlichungstag und Bekanntmachung des Hinweises auf die Patenterteilung: **11.02.2009**
(21) Anmeldenummer: 02027058.3
(22) Anmeldetag: 03.12.2002
(51) Int. Cl.: C12N 9/80, C12N 15/55, C12N 15/11, C12N 5/10, C12N 1/20

(54) **D-Amidase aus Variovorax**
D-amidase from Variovorax
Variovorax Amidase D

(30) Priorität: 06.12.2001 DE 10160066
(43) Veröffentlichungstag der Anmeldung: 11.06.2003
(73) Patentinhaber: Evonik Degussa GmbH, 45128 Essen (DE)
(72) Erfinder: Verseck, Stefan, Dr., 63452 Hanau (DE); Drauz, Karlheinz, Prof., 63579 Freigericht (DE); Bommarius, Andreas, Prof., 303 Atlanta, GA (US); Kula, Maria-Regina, Prof., 81477 München (DE); Krieg, Lutz, 52428 Jülich (DE); Slusarczyk, Heike, Dr., 52531 Übach-Palenberg (DE); Ansorge, Marion, Dr., 52076 Aachen (DE)

(56) Entgegenhaltungen:
- EP-A- 0 334 358
- WO-A-97/12964
- HAYASHI TAKAHIKO ET AL: "Characterization and cloning of an enantioselective amidase from Comamonas acidovorans KPO-2771-4." JOURNAL OF FERMENTATION AND BIOENGINEERING, Bd. 83, Nr. 2, 1997, Seiten 139-145, XP009009228 ISSN: 0922-338X -& DATABASE EMBL [Online] 2. Mai 1997 (1997-05-02) "Comamonas acidovorans derived amidase gene" Database accession no. AAT47765 XP002237979
- OZAKI AKIO ET AL: "A D-amidase constitutive mutant from Arthrobacter sp. NJ-26." BIOSCIENCE BIOTECHNOLOGY AND BIOCHEMISTRY, Bd. 57, Nr. 3, 1993, Seiten 520-521, XP009009243 ISSN: 0916-8451
- LEADBETTER JARED R ET AL: "Metabolism of acyl-homoserine lactone quorum-sensing signals by Variovorax paradoxus" JOURNAL OF BACTERIOLOGY, WASHINGTON, DC, US, Bd. 182, Nr. 24, Dezember 2000 (2000-12), Seiten 6921-6926, XP002208346 ISSN: 0021-9193

## Beschreibung

Die vorliegende Erfindung betrifft eine Amidase aus dem Organismus der Gattung *Variovorax*, sowie die dieses Enzym codierenden Nukleinsäuren und Vehikel, aufweisend diese Nukleinsäuren.

Insbesondere richtet sich die Erfindung auf eine Amidase aus *Variovorax paradoxus* 19-3 DSM 14468, sowie die dieses Enzym codierenden Nukleinsäuren sowie den Stamm an sich.

Amidasen bzw. Amidohydrolasen werden nach dem E.C.-System in zwei Unterklassen, E.C. 3.5.1.1 bis 3.5.1.77 und E.C. 3.5.2.1 bis 3.5.2.14 eingeteilt. Vertreter der ersten Unterklasse sind z. B. die Asparaginase (E.C. 3.5.1.1), Urease (E.C. 3.5.1.5) und die hier näher betrachtete Acyl-amid Amidohydrolase, (E.C. 3.5.1.4).

Innerhalb der Mikroorganismen ist die Acylamid Amidohydrolase weit verbreitet und kommt unter anderem in Spezies wie Corynebacteria, Pseudomonaden, Bacilli, Brevibacteria, Rhodococcen und Alcaligenes vor. Es sind meist induzierbare Enzyme, deren Spezifität von Organismus zu Organismus stark variert (Maestracci, M.; Bui, K.; Thiéry, A.; Arnaud, A.; Galzy, P. (1988), The Amidases from a Brevibacterium Strain: Study and Applications, Adv. Biochem. Eng. 36, 69-115).

Die Enzyme aus *Mycobacterium neoaurum* ATCC 25795 (Hermes, H. F. M.; Tandler, R. F.; Sonke, T.; Dijkhuizen, L.; Meijer, E. M. (1994), Purification and Characterization of an L-Amino Amidase from Mycobacterium neoaurum ATCC 25795, Appl. Environ. Microbiol. 60, 153-159) und *Pseudomonas putida* ATCC 12633 (Hermes, H. F. M.; Sonke, T.; Peters, P. J. H.; van Balken, J, A. M.; Kamphuis, J.; Dijkhuizen, L.; Meijer, E. M. (1993), Purification and Characterization of an L-Aminopeptidase from Pseudomonas putida ATCC 12633, Appl. Environ. Microbiol. 59, 4330-4334) sind besonders zur Hydrolyse von L-Aminosäurenamide von technischer Bedeutung. Beide Enzyme zeigen eine höhere Affinität zu N-verzweigten Aminosäureamiden bzw. Dipeptiden und werden somit als Aminopeptidasen (E.C. 3.4.) klassifizert. Die L-Aminopeptidase aus *Pseudomonas putida* ATCC 12633 wird zur stereospezifischen Spaltung eines D,L-Phenylglycinamid-Gemisches in D-Phenylglycinamid und L-Phenylglycin eingesetzt. Ein durch die DSM entwickelter Prozeß nutzt ganze Zellen von *Pseudomonas putida* zur Herstellung reiner D- und L-Aminosäuren aus D,L-Aminosäureamiden (Kamphuis, J.; Boesten, W. H. J.; Broxterman, Q. B.; Hermes, H. F. M.; Balkan van, J. A. M.; Meijer, E. M.; Shoemaker, H. E. (1990), New developments in the chemo-enzymatic production of amino acids, Adv. Biochem. Eng. Biotechnol. 42, 133-186).

Weiterhin wurde durch die Schering AG ein Verfahren zur Herstellung von L-Aminosäuren und Aminosäurenamiden aus D,L-α-Aminonitrilen patentiert (Klages, U.; Weber, A. (1988), Verfahren zur Herstellung von L-Aminosäuren und Aminosäureamiden, DE 3 816 063 A1; WO 8 910 969). Bei dieser Biotransformation mit ganzen Zellen werden zunächst mit A-*cinetobacter calcoaceticus DSM 3875* die D,L-Aminonitrile zu D,L-Aminosäurenamide hydrolysiert. Mit einer L-Aminosäure-Amidase und einer Aminosäureamid-Racemase in *Arthrobacter sp. ATCC 31652* oder *Corynebacterium sp. ATCC 31662* ist grundsätzlich eine vollständige Umsetzung zur L-Aminosäure möglich.

Durch die Entdeckung von Aminosäureamid-Racemasen in *Pseudomonas putida* und *Rhodococcus sp.* konnte für Novo Industri A/S, Dänemark und Stamicarbon B.V., Niederlande ein Verfahren zur Racemisierung von Aminosäureamiden und Hydrolyse durch eine L- oder D-Amidase zur entsprechenden Aminosäure patentiert werden (Godtfredsen, S. E.; Clausen, K.; Ingvorsen, K.; Hermes, H. F.; Van Balken, J. A.; Meijer, E. M. (1989), EP 0 307 023; WO 8 901 525). Eine D-Amidase-Aktivität wurde hier in *Pseudomonas putida* NCIB 40042 und *Rhodococcus sp.* NCIB 40041 beschrieben.

Die Entdeckung einer weiteren Aminosäureamid-Racemase bei *Klebsiella oxytoca* beschreiben Hermes und Mitarbeiter (Hermes, H. F. M.; Peeters, W. P.; Peters, P. J. (1990), EP 0 383 403).

Weitere Amidasen mit einer D-Spezifität bezüglich Aminosäureamide sind *in Comamonas acidovorans* KPO-2771-4 (Hayashi, T.; Yamamoto, K.; Matsuo, A.; Otsubo, K.; Muramatsu, S.; Matsuda, A.; Komatsu, K.-I. (1997), Characterization and Cloning of an Enantioselective Amidase from Comamonas acidovorans KPO-2771-4, J. Ferment. Bioeng. 83, 139-145) und in zwei Stämmen von *Ochrobactrum anthropi*, SCRC C1-38 (Asano, Y.; Kato, Y.; Yamada, A.; Kondo, K. (1992) Structural Similarity of D-Aminopeptidase to Carboxypeptidase DD and β-Lactamases, Biochem. 31, 2316-2328; Asano, Y.; Nakazawa, A.; Kato, Y.; Kondo, K. (1989), Properties of a Novel D-Stereospecific Aminopeptidase from Ochrobactrum anthropi, J. Biol., Chem. 264, 14233-14239) und SCRC-SV3 beschrieben (Komeda, H. und Asano, Y. (2000), Gene cloning, nucleotide sequencing, and purification and characterisation of the D-stereospecific amino-acid amidase from Ochrobactrum anthropi SV3, Eur. J. Biochem. 267, 2028-2035; Asano, Y.; Mori, T.; Hanamoto, S.; Kato, Y.; Nakazawa, A. (1989), A New D-Stereospecific Amino Acid Amidase From Ochrobactrum anthropi, Biochem. Biophys. Res. Commun. 162, 470-474).

Trotzdem herrscht weiterhin ein Bedarf an D-Amidasen, zumal deren Substratspektren sich nicht 100%ig decken und durch das Auffinden neuer Enzyme vormals schlecht umsetzbare Substrate nunmehr unter ökonomisch vorteilhaften Gesichtspunkten im technischen Maßstab hergestellt werden könnten.

Die Aufgabe der vorliegenden Erfindung bestand daher in der Bereitstellung neuer D-Amidasen.

Die Aufgabe wird anspruchsgemäß gelöst. Anspruch 1 bezieht sich auf eine D-Amidase aus der Gattung *Variovorax paradoxus*. Anspruch 2 stellt die diese Enzyme codierenden Nukleinsäuren unter Schutz. Anspruch 3 wiederum betrifft Vehikel, welche die erfindungsgemäßen Nukleinsäuren aufweisen, Anspruch 4 schützt bevorzugte Primer. Anspruch 5 und 6 richten sich auf bestimmte Verwendungen der erfindungsgemäßen D-Amidasen bzw. erfindungsgemäßen Nukleinsäuren. Anspruch 7 ist auf Ganzellkatalysatoren gerichtet. Anspruch 8 schützt den neuen Organismus *Variovorax paradoxus* 19-3 DSM 14468.

Dadurch, daß man eine D-Amidase aus Variovorax paradoxus DSM 14468 Verfügung stellt, besitzt man die Möglichkeit, die gestellte Aufgabe vorteilhaft zu lösen.

Die erfindungsgemäße D-Amidase ist in der Lage, die Hydrolyse eines breiten Spektrums von Carbonsäureamiden, in die entsprechenden Carbonsäuren zu katalysieren. Dabei werden racemische Gemische an z.B. Aminosäureamiden z.T. streng D-selektiv umgewandelt. Mit der vorliegenden Erfindung ist es überraschenderweise möglich, enantiomerenangereichertes D-tert-Leucin aus racemischem tert-Leucinamid in einer für einen technischen Prozeß ausreichenden turn-over-frequency zu gewinnen.

In einer nächsten Ausgestaltung beschäftigt sich die Erfindung mit Nukleinsäuren codierend für eine erfindungsgemäße D-Amidase.

Trotz der Kultivierbarkeit der Variovorax-Stämme und einfachen Zugänglichkeit des Enzyms über chromatographische Methoden gelangt man durch die Angabe der Nukleinsäuren codierend für eine erfindungsgemäße D-Amidase in weiterhin bevorzugter Art und Weise zu Substanzen, welche es erlauben, die für einen enzymatisch-technischen Prozeß zur Erzeugung von enantiomerenangereicherten Verbindungen notwendigen Enzyme in ausreichender Menge über rekombinante Techniken sicherzustellen. Es ist mit den Nukleinsäuren möglich, die Enzyme aus schnell wachsenden Wirtsorganismen in hohen Ausbeuten zu gewinnen. Außerdem sind die erfindungsgemäßen Gensequenzen zur Erzeugung von verbesserten Mutanten zu verwenden.

In einer nächsten Ausgestaltung bezieht sich die Erfindung auf Plasmide oder Vektoren aufweisend eine oder mehrere der erfindungsgemäßen Nukleinsäuren.

Als Plasmide oder Vektoren kommen im Prinzip alle dem Fachmann für diesen Zweck zur Verfügung stehenden Ausführungsformen in Frage. Derartige Plasmide und Vektoren können z. B. von Studier und Mitarbeiter (Studier, W. F.; Rosenberg A. H.; Dunn J. J.; Dubendroff J. W.; (1990), Use of the T7 RNA polymerase to direct expression of cloned genes, Methods Enzymol. 185, 61-89) oder den Broschüren der Firmen Novagen, Promega, New England Biolabs, Clontech oder Gibco BRL entnommen werden. Weiter bevorzugte Plasmide und Vektoren können gefunden werden in: Glover, D. M. (1985), DNA cloning: a practical approach, Vol. I-III, IRL Press Ltd., Oxford; Rodriguez, R.L. und Denhardt, D. T (eds) (1988), Vectors: a survey of molecular cloning vectors and their uses, 179-204, Butterworth, Stoneham; Goeddel, D. V. (1990), Systems for heterologous gene expression, Methods Enzymol. 185, 3-7; Sambrook, J.; Fritsch, E. F. und Maniatis, T. (1989), Molecular cloning: a laboratory manual, 2nd ed., Cold Spring Harbor Laboratory Press, New York Plasmide, mit denen das die erfindungsgemäße Nukleinsäure aufweisende Genkonstrukt in ganz bevorzugter Weise in den Wirtsorganismus kloniert werden kann, sind: pUC18 (Roche Biochemicals), pKK-177-3H (Roche Biochemicals), pBTac2 (Roche Biochemicals), pKK-233-3 (Stratagene) oder pET (Novagen).

Gleichfalls ist die Erfindung auf Mikroorganismen aufweisend die erfindungsgemäßen Nukleinsäuren gerichtet.

Der Mikroorganismus, in den die Nukleinsäuren kloniert werden, dient zur Vermehrung und Gewinnung einer ausreichenden Menge des rekombinanten Enzyms. Die Verfahren hierfür sind dem Fachmann wohlbekannt (Sambrook, J.; Fritsch, E. F. und Maniatis, T. (1989), Molecular cloning: a laboratory manual, 2nd ed., Cold Spring Harbor Laboratory Press, New York). Als Mikroorganismen können im Prinzip alle dem Fachmann für diesen Zweck in Frage kommenden Organismen wie z.B. Hefen wie *Hansenula polymorpha*, *Pichia sp., Saccharomyces cerevisiae,* Prokaryonten, wie *E. coli, Bacillus subtilis* oder Eukaryonten, wie Säugerzellen, Insektenzellen herangezogen werden. Vorzugsweise sind *E. coli*-Stämme für diesen Zweck zu benutzen. Ganz besonders bevorzugt sind: *E. coli* XL1 Blue, NM 522, JM101, JM109, JM105, RR1, DH5α, TOP 10⁻ oder HB101. Plasmide, mit denen das die erfindungsgemäße Nukleinsäure aufweisende Genkonstrukt vorzugsweise in den Wirtsorganismus kloniert wird, sind weiter oben angegeben.

Mitumfaßt von der Beschreibung sind auch Nukleinsäuren, welche unter stringenten Bedingungen mit den erfindungsgemäßen einzelsträngigen Nukleinsäuren oder deren komplementären einzelsträngigen Nukleinsäuren hybridisieren. Als solche ist z.B. die Gensonde gemäß See. 5 anzusehen.

Der Ausdruck "unter stringenten Bedingungen" wird hierin wie bei Sambrook et al. (Sambrook, J.; Frisch, E. F. und Maniatis, T. (1989), Molecular cloning: a laboratory manual, 2nd ed., Cold Spring Harbor Laboratory Press, New York) beschrieben, verstanden. Bevorzugt liegt eine stringente Hybridisierung gemäß der vorliegenden Beschreibung vor, wenn nach Waschen für eine Stunde mit mit 0,2 x SSC und 0,1 % SDS bei 50 °C, bevorzugter bei 55 °C, mehr bevorzugt bei 62 °C und am meisten bevorzugt bei 68 °C noch ein positives Hybridisierungssignal beobachtet wird.

Ein folgender Aspekt der Erfindung richtet sich auf Primer zur Herstellung der erfindungsgemäßen Gensequenzen mittels aller Arten von PCR. Mitumfaßt sind die Sense- und Antisense-Primer codierend für die entsprechenden Aminosäuresequenzen, bzw. komplementären DNA-Sequenzen gemäß der Sequnzein 2 < ... >. Geeignete Primer können prinzipiell nach dem Fachmann bekannten Verfahren gewonnen werden. Das Auffinden der erfindungsgemäßen Primer erfolgt durch Vergleich mit bekannten DNA-Sequenzen oder durch Übersetzung der ins Auge gefaßten Aminosäuresequenzen in das bevorzugte Codon des betrachteten Organismus (z.B. für Streptomyces: Wright F. und Bibb M. J. (1992), Codon usage in the G+C-rich Streptomyces genome, Gene 113, 55-65). Gemeinsamkeiten in der Aminosäuresequenz von Proteinen von sogenannten Superfamilien sind hierfür ebenfalls von Nutzen (Firestine, S. M.; Nixon, A. E.; Benkovic, S. J. (1996), Threading your way to protein function, Chem. Biol. 3, 779-783). Weitere Informationen diesbezüglich können gefunden werden in Gait, M. J. (1984), Oligonucleotide synthesis: a practical approach, IRL Press Ltd., Oxford; Innis, M. A.; Gelfound, D. H.; Sninsky, J. J. und White, T.J. (1990), PCR Protocols: A guide to methods and applications, Academic Press Inc., San Diego. Die Primer sind folgende:
AAH-N1: 5' GTS GGC CGS CGS ATC CAG CAG AAG GA 3' (Sequenz 3)
AAH-C1: 5' GGG ATS CGG ATC GAG CCG CCS GTS TC 3' (Sequenz 4)

Die Bezeichnung S steht für G + C in der Sequenz von AAH-N1 und AAH-C1 (IUB Gruppencode zur Identifizerung von Redundanzen).
AAH-GW-F1: 5' GCG TCA CGC CGC CGG TCA ATC CGT GGA A 3' (Sequenz 6)
AAH-GW-F2: 5' GGC GCA CTG GTC GGG TGC CTC GTC GA 3' (Sequenz 7)
AAH-GW-R1: 5' CAG CGC GTG TTC GGC CAT CAC GAT CAC ATA 3' (Sequenz 8)
AAH-GW-R2: 5' CTC TTG AGC GCG CCG TCG ACC TTC TCG A 3' (Sequenz 9)
AAH-K-N2: 5' CTG GTC ATC AAG CGC GGC CAG ATC GGC 3' (Sequenz 10)
AAH-K-C2: 5' GAT CGG CCG ACA GCC GAT TGG CCA GC 3' (Sequenz 11)
AAH-N-EcoRI: 5' CCG GAA TTC ATG AGC AAC GAA CTG CAT TAC CT 3' (Sequenz 12)
AAH-C-HindIII: 5' ATC CCA AGC TTT TAC AGC ACC GGA TGC CG 3' (Sequenz 13)

Die vorliegende Erfindung beschäftigt sich ebenfalls mit der Verwendung der erfindungsgemäßen D-Amidasen zur Herstellung von Carbonsäuren oder ggf. chiralen enantiomerenangereicherten organischen Verbindungen, wie z.B. Aminosäuren.

Weiterhin eigenen sich die erfindungsgemäßen Nukleinsäuren, die für die betrachteten D-Amidasen codieren, bevorzugt zur Herstellung von Ganzzellkatalysatoren.

Ebenfalls Gegenstand der Erfindung ist ein Ganzzellkatalysator aufweisend ein kloniertes Gen für eine Nitrilhydratase und ein kloniertes Gen für eine erfindungsgemäße Amidase und optional ein kloniertes Gen für eine α-Aminonitril-Racemase, eine Cyanhydrin-Racemase, eine α-Hydroxycarbonsäure-Racemase oder eine (α- bzw, β-) Aminosäureamid-Racemase (Hermes, H. F. M.; Peeters, W. P.; Peters, P. J. (1990), EP 0 383 403; sowie WO 8 901 525; Wilms, L.; Bartsch, K. (1995), EP 0 690 133; Klages, U.; Weber, A (1989), WO 8 910 969)

Der erfindungsgemäße Ganzzellkatalysator weist eine D-Amidase aus *Variovorax paradoxus,* DSM 14468 auf. Die Herstellung eines solchen Organismus ist dem Fachmann geläufig (Farwick, M.; London, M.; Dohmen, J.; Dahlems, U.; Gellissen, G.; Strasser, A. W.; DE19920712).

Der Vorteil eines derartigen Organismus ist die gleichzeitige Expression beider Enzymsysteme, womit nur noch ein rec-Organismus für die Reaktion angezogen werden muß. Um die Expression der Enzyme im Hinblick auf ihre Umsetzungsgeschwindigkeiten abzustimmen, können die entsprechend codierenden Nukleinsäurefragmente auf unterschiedlichen Plasmiden mit unterschiedlichen Kopienzahlen untergebracht werden und/oder unterschiedlich starke Promotoren für eine unterschiedlich starke Expression der Gene verwendet werden.

Bei derart abgestimmten Enzymsystemen tritt vorteilhafterweise eine Akkumulation einer ggf. inhibierend wirkenden Zwischenverbindung nicht auf und die betrachtete Reaktion kann in einer optimalen Gesamtgeschwindigkeit ablaufen. Dies ist dem Fachmann jedoch hinlänglich bekannt (Gellissen, G.; Piontek, M.; Dahlems, U.; Jenzelewski, V.; Gavagan, J. W.; DiCosimo, R.; Anton, D. L.; Janowicz, Z. A. (1996), Recombinant Hansenula polymorpha as a biocatalyst. Coexpression of the spinach glycolate oxidase (GO) and the S. cerevisiae,catalase T (CTT1) gene, Appl. Microbiol. Biotechnol. 46, 46-54; Farwick, M.; London, M.; Dohmen, J.; Dahlems, U.; Gellissen, G.; Strasser, A. W.; DE19920712).

Die erfindungsgemäßen Nukleinsäuren lassen sich also vorzugsweise zur Herstellung von rec-D-Amidasen einsetzen. Durch rekombinante Techniken, die dem Fachmann hinlänglich bekannt sind (s.u.), gelangt man zu Organismen, welche in der Lage sind, das betrachtete Enzym in für einen technischen Prozeß ausreichender Menge zur Verfügung zu stellen. Die Herstellung der erfindungsgemäßen rec-Enzyme erfolgt nach dem Fachmann bekannten gentechnologischen Verfahren (Sambrook, J.; Fritsch, E. F. und Maniatis, T. (1989), Molecular cloning: a laboratory manual, 2nd ed., Cold Spring Harbor Laboratory Press, New York; Balbas, P. und Bolivar, F. (1990), Design and construction of expression plasmid vectors in E.coli, Methods Enzymol. 185, 14-37; Rodriguez, R.L. und Denhardt, D. T (eds) (1988), Vectors: a survey of molecular cloning vectors and their uses, 205-225, Butterworth, Stoneham). Bezüglich der allgemeinen Vorgehensweise (PCR, Klonierung, Expression etc.) sei auch auf folgende Literatur und das dort zitierte verwiesen: Universal GenomeWalker^{™} Kit User Manual, Clontech, 3/2000 und dort zitierte Literatur; Triglia T.; Peterson, M. G. und Kemp, D.J. (1988), A procedure for in vitro amplification of DNA segments that lie outside the boundaries of known sequences, Nucleic Acids Res. 16, 8186; Sambrook, J.; Fritsch, E. F. und Maniatis, T. (1989), Molecular cloning: a laboratory manual, 2nd ed., Cold Spring Harbor Laboratory Press, New York; Rodriguez, R.L. und Denhardt, D. T (eds) (1988), Vectors: a survey of molecular cloning vectors and their uses, Butterworth, Stoneham.

Gegenstand der Erfindung ist ebenfalls der Mikroorganismus *Variovorax paradoxus* 19-3 nach dem Budapester Vertrag hinterlegt bei der DSMZ am 22.08.2001 unter der Nummer 14468.

Die D-Amidase aus *Variovorax paradoxus* wird nach dem E.C.-System als eine Acylamid Amidohydrolase (E.C.3.5.1.4) klassifizert. Die vollständige Gensequenz der D-Amidase aus Va*riovorax paradoxus* ist in Seq. 1 angegeben. Ein Genabschnitt der D-Amidase wurde durch eine PCR-Reaktion mit genomischer DNA aus *Variovorax paradoxus* erhalten. Ein Primer, AAH-N1 (Seq. 3), konnte aus dem N-Terminus der ermittelten Aminosäuresequenz erhalten werden. Der zweite Primer, AAH-C1 (Seq. 4), wurde aufgrund der Sequenzähnlichkeit des N-Terminus mit Vertretern der sogenannten Amidase-Familie aus der Consensus Region abgeleitet. Mit diesen zwei Primern wurde ein 509 bp großes Fragment des D-Amidase-Gens mittels PCR-Technik amplifizert, welches grundsätzlich als Gensonde eingesetzt werden kann. Seine Abfolge an Basenpaaren ist in Sequenz 5 dargestellt.

Anhand dieses Fragments wurden weitere genspezifische Primer konstruiert, die für die Methode des Universal Genome-Walker^{™}-Kit (Universal GenomeWalker^{™} Kit User Manual, Clontech, 3/2000 und dort zitierte Literatur) der Firma Clontech zum Auffinden des gesamten Gens benötigt wurden (Seq. 6 bis 9).

Nach der Methode des Universal GenomeWalker^{™}-Kit wurde zunächst genomische DNA aus *Variovorax paradoxus* jeweils mit den Restriktionsenzymen EcoRV, PvuII, ScaI und StuI geschnitten. Die Ansätze mit den erhaltenen DNA-Fragmenten wurden mit dem beigefügten GenomeWalker^{™} Adaptor ligiert, für den zwei Primer vorliegen.

Mit diesen Ansätze als Template wurde mit Hilfe einer der aufgeführten genspezifischen Primer AAH-GW-F1 (Seq. 6) oder AAH-GW-R1 (Seq. 8) und eines Adaptorprimers eine PCR-Reaktion in beide Richtungen durchgeführt. Eine sogenannte Nested-PCR (Universal GenomeWalker^{™} Kit User Manual, Clontech, 3/2000 und dort zitierte Literatur) mit den Primern AAH-GW-F2 (Seq. 7) oder AAH-GW-R2 (Seq. 8) und eines Adaptorprimers soll die Bildung von unspezifischen PCR-Produkten verhindern. Mit den erhaltenen PCR-Fragmenten von bis zu 3500 bp (downstream) und 1800 bp (upstream) war die komplette Gensequenz der D-Amidase zugänglich.

Für die weiteren Arbeiten und für eine Überprüfung der Gensequenz wurde ausgehend von genomischer DNA und folgenden Primern (Seq. 10 und 11) das gesamte D-Amidase-Gen mit 92 bp vor dem Startcodon und 80 bp nach dem Stopcodon in drei parallelen Ansätzen mit zwei verschiedenen DNA-Polymerasen mit Proofreading (VentR®, New England Biolabs und Herculase®, Stratagene) amplifiziert.

Die erhaltenen PCR-Produkte wurden blunt-end in den Vector pUC18 ligiert und in E. coli XL1 Blue transformiert. Ausgehend von Plasmid-DNA konnte somit die D-Amidase-Sequenz (Seq. 1) sichergestellt und überprüft werden (Fa. Sequiserve, Vaterstetten, Germany).

Durch eine PCR-Reaktion wurden für die Expression der D-Amidase eine EcoRI- am 5'-Ende und eine HindIII-Schnittstelle am 3'-ende eingeführt (Seq. 12 und 13) und das PCR-Produkt in den pBTAC-Vektor ligiert und in *E. coli* JM 101 transformiert.

In einem ersten Expressionsversuch konnte eine spez. Akt. zwischen 80 und 210 mU/mg für Dri-Tle-NH₂ im Rohextrakt bestimmt werden. Im Vergleich zur spez. Akt. in *Variovorax paradoxus* zwischen 20 und 30 mU/mg im Rohextrakt konnte eine deutliche Überexpression erreicht werden. Eine Analyse der löslichen und unlöslichen Fraktionen der Rohextrakte mittels SDS-PAGE ergab, daß ein Großteil an exprimierter D-Amidase in unlöslicher Form, sogenannten inclusion bodies, im Zellpellet vorliegt.

Die Aufreinigung der Amidase erfolgt in drei Chromatographieschritten, wobei meist nach dem 2. Schritt nur noch ein sehr geringer Anteil an Fremdprotein vorliegt:
1. Ionenaustauschchromatographie: Q-Sepharose FF (Pharmacia)
2. Hydrophobe Interaktionschromatographie: Butyl-Sepharose 4 FF (Pharmacia)
3. Gelfiltration: Superdex 200 PG (Pharmacia)

Für die ersten zwei Aüfreinigungsschritte läßt sich eine Ausbeute von 88 % berechnen, wobei eine spez. Akt. von 0,68 U/mg für DL-Tle-NH₂ alls Substrat vorliegt. Für die homogen aufgereinigte D-Amidase aus *Variovorax paradoxus* ergab sich eine spez. Akt. von 1, 4 U/mg für DL-Tle-NH₂.

Die Eignung der Amidase wurde in folgenden Versuchen belegt:

### 1. Säureamide:

Die Aktivitätsmessung erfolgte durch Bestimmung der Ammoniumionen mittels der Glutamat-Dehydrogenase (Bergmeyer, H. U., and Beutler, H.-O. (1985), Ammonia. In: Methods of Enzymatic Analysis. VCH-Verlag, 3rd Edition, Vol. 8: 454-461, Weinheim). Das verwendete Enzym war eine angereinigte D-Amidase nach dem 2. Chromatographieschritt mit einer spez. Akt. von 0,51 U/mg. Die Substrate wurden im Enzymtest 40 mM eingesetzt, außer Bernsteiasäurediamid, Adipinsaurediamid jeweils 10 mM und Benzylamid 20 mM. Da die Aktivitäten nur bei jeweils einer Substratkonzen-tration bestimmt wurden, sind relative Aktivitäten angegeben.

**Tab. 1: Relative Enzymativitäten für verschiedene Säureamide bzgl. DL-Tle-NH₂**

| **Substrat** | **relative Akt. [U/mg]** | **Aktivität bezgl-Tle-NH₂** |
|---|---|---|
| DL-Tle-NH₂ | 0,51 | 1 |
| Formamid | 0,061 | 0,13 |
| Acetamid | 2,1 | 4,0 |
| Propionamid | 12 | 26 |
| Butyramid | 17 | 37 |
| Isobutyramid | 15 | 33 |
| Valerianamid | 16 | 36 |
| Acetessigsäureamid | 58 | 130 |
| Malonsäurediamid | 1,5 | 2,9 |
| Bernsteinsäurediamid | 1,1 | 2,1 |
| Adipinsäurediamid | 7,0 | 14 |
| Acrylamid | 4,0 | 7,7 |
| Benzylamid | 2,6 | 5,2 |
| Nicotinamid | 8,5 | 19 |

### 2. Aminosäureamide und α-Hxdroxycarbonsäureamide

Für Prolinamid, und die α-Hxdroxycarbonsäureamide Milchsäureamid (Lac-NH2) und 2-Hydroxy-4-methylmercaptobuttersäureamid (MHA-NH2), das methioninanaloge α-Hxdroxycarbonsäureamid, ist die Bestimmung des Produktes mit der bisherigen HPLC-Analytik nicht möglich. Für diese Substrate wurde die Aktivität anhand der NH₄⁺-Bestimmung ermittelt. Das verwendete Enzym war eine angereinigte D-Amidase nach dem 2.Chromatographieschritt mit einer spez. Akt. von 0,56 U/mg. Alle Substrate wurden im Enzymtest 40 mM eingesetzt. In der 4. Spalte wurde für einen weiteren vergleich die Aktivität für das geringere Enantiomer gleich 1 gesetzt und daraus eine relative Aktivität für das bevorzugte Enantiomer berechnet.

**Tab. 2: Enzymaktivitäten für Amino- und Hxdroxysäureamide**

| **Substrat** | **relative Akt. [U/mg]** | **Aktivität bezgl- Tle-HH₂** | **rel. Aktivität bezgl-D und L** |
|---|---|---|---|
| DL-Tle-NH₂ | 0,56 | 1 | - |
| D-Ala-NH₂ | 23 | 44 | 3,0 |
| L-Ala-NH₂ | 7,7 | 15 | 1 |
| D-Leu-NH₂ | 220 | 400 | 15 |
| L-Leu-NH₂ | 15 | 26 | 1 |
| D-Val-NH₂ | 6,9 | 13 | 11 |
| L-Val-NH₂ | 0,59 | 1,2 | 1 |
| L-Ile-NB₂ | 0,17 | 0,35 | - |
| D-Phe-NH₂ | 500 | 890 | 4,6 |
| L-Phe-NH₂ | 110 | 190 | 1 |
| L-Tyr-NH₂ | 47 | 100 | - |
| DL-Trp-NH₂ | 86 | 160 | 2,5 |
| L-Trp-NH₂ | 34 | 62 | 1 |
| DL-Met-NH₂ | 130 | 230 | 2,7 |
| L-Met-NH₂ | 48 | 86 | 1 |
| D-Pro-NH₂ | 12 | 24 | 1 |
| L-Pro-NH₂ | 28 | 56 | 2,3 |
| D-Lac-NH₂ | 29 | 59 | 1 |
| L-Lac-NH₂ | 33 | 66 | 1,1 |
| DL-MHA-NH₂ | 82 | 150 | - |

Weiterhin wurde die Eignung der D-Amidase zur kinetischen Racematspaltung von DL-Tle-NH₂ gemäß nachstehendem Schema untersucht.

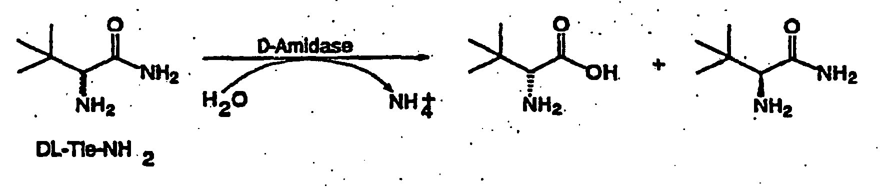

Um die Eignung für eine kinetische Racematspaltung von DL-Tle-NH₂ zu beurteilen, wurde die Enantioselektivität in Abhängigkeit vom Umsatz bestimmt. Bereits mit angereinigtem Enzym nach dem 1.Chromatographieschritt (Ionenaustauschchromatographie: Q-Sepharose FF (Pharmacia)) wurde bei einem Umsatz größer als 45% Enantioselektivitäten von - 96,6% bezüglich D-Tle bestimmt. Mit nahezu homogenem Enzym wurden im Bereich von 45 bis 50% Umsatz ee-Werte zwischen 99,4 und 98,6% gemessen. Für beide Versuchsreihen berechnet sich somit ein E-Wert (Straathof, A. J. J. und Jongejan, J. A. (1997). The enantiomeric ratio: origin, determination and prediction, Enzyme Microb. Technol. 21, 559-571) größer 100. Dies ist für die Racematspaltungmit einer erwünschten nahezu vollständiger Umsetzung des D-Enantiomers eine sehr gute Grundlage.

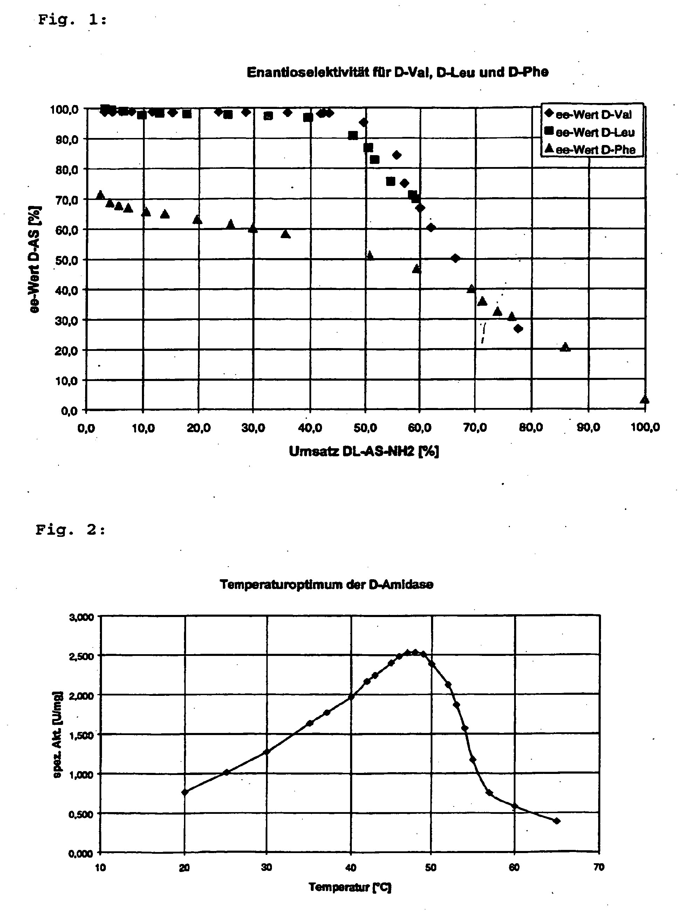

Weiterhin wurden DL-Valin-, DL-Leucin- und DL-Phenylalaninamid diesbezüglich getestet. Hierzu wurde mit angereinigtem Enzym aus *Variovorax paradoxus* 19-3 nach dem 1.Chromatographieschritt die Enantioselektivität in Abhängigkeit vom Umsatz bestimmt. Fig. 1 zeigt die Enantioselektivität für D-Val, D-Leu und D-Phe in Abhängigkeit vom Umsatz.

In der folgenden Tabelle sind exemplarisch einige Meßwerte, insbesonders im Bereich von 50 % Umsatz aufgeführt und die daraus berechneten E-Werte.

**Tab. 3: Enantioselektivitäten für D-Val, D-Leu und D-Phe**

| **DL-Val-NH₂** | | |
|---|---|---|
| Umsatz [%] | ee D-Val [%] | E-Wert |
| 3 | 98,9 | 190 |
| 15 | 98,7 | 180 |
| 29 | 98,5 | 200 |
| 42 | 98,1 | 220 |
| 50 | 95,1 | 140 |
| 57 | 75,0 | 56 |

| **DL-Leu NH₂** | | |
|---|---|---|
| Umsatz [%] | ee D-Leu [%] | E-Wert |
| 4 | 99,6 | 470 |
| 18 | 98,1 | 130 |
| 33 | 97,3 | 120 |
| 40 | 96,7 | 114 |
| 48 | 90,8 | 53 |
| 51 | 86,7 | 41 |

| **DL-Phe-NH₂** | | |
|---|---|---|
| Umsatz [%] | ee D-Phe [%] | E-Wert |
| 4 | 68,8 | 5,6 |
| 14 | 65,1 | 5,2 |
| 26 | 61,5 | 5,1 |
| 36 | 58,3 | 5,2 |
| 51 | 50,9 | 5,1 |

Im Bereich von 40 bis 50 % Umsatz ergeben sich Enantioselektivitäten für D-Val im Bereich von 98,1 bis 95,1 %, für D-Leu im Bereich von 96,7 bis 90,8 % und für D-Phe im Bereich von 55%. Für das racemische Val-NH₂ und Leu-NH₂ berechnen sich mittlere E-Werte größer 100 und für Phe-NH₂ ein E-Wert von 5.

Im Hinblick auf eine Racematspaltung mit einer erwünschten nahezu vollständigem Umsetzung des D-Enantiomers ist somit die D-Amidase, für DL-Val-NH₂ und DL-Leu-NH₂ grundsätzlich sehr gut geeignet.

### Erweiterung des Substratspektrums:

Die Racematspaltung von N-formylierten Aminosäurenamide wurde mittels der erfindungsgemäßen D-Amidase überprüft.

Das verwendete Enzym war eine angereinigte D-Amidase nach dem 1. Chromatographieschritt. Die Aktivitätsmessung erfolgte durch Bestimmung des NH₄⁺-Ionen mittels der Glutamat-Dehydrogenase (Bergmeyer, H.,U., and Beutler, H.-O. (1985) Ammonia. In: Methods of Enzymatic Analysis. VCH-Verlag, 3rd Edition, Vol. 8: 454-461, Weinheim). Die folgenden Substrate wurden im Enzymtest 10 mM eingesetzt.

**Tab. 4: Relative Enzymaktivitäten für N-formyliertes Val-NH₂**

| **Substrat** | **relative Akt. [mU/mg]** | **Aktivität bezgl-Tle-NH₂ [%]** |
|---|---|---|
| DL-Tle-NH₂ | 200 | 100 |
| DL-Formyl-Val-MH₂ | 670 | 280 |

Interessant erscheint die Umsetzung des racemischen N-Formyl-Valinamids, daß um den Faktor 2,8 besser umgesetzt wird als DL-Tle-NH₂.

**Tab. 5: Vergleich der Enzymaktivitäten Valinamid und N-Formyl-Valinamid**

| **Substrat** | **relative Akt. [mU/mg]** | **Aktivität bezgl-Tle-NH₂ [%]** |
|---|---|---|
| DL-Val-NH₂ | 870 | 100 |
| DL-Formyl-Val-NH₂ | 670 | 77 |

### Temperatur- und pH-Optimum der D-Amidase:

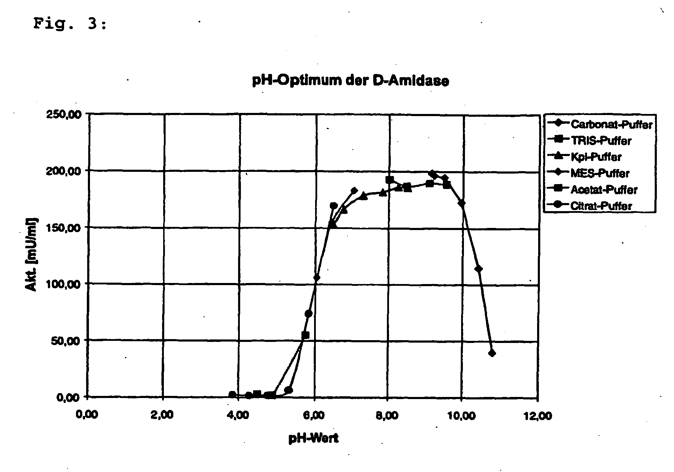

In Fig. 2 und 3 ist die Abhängigkeit der Aktivität von der Temperatur und dem pH-Wert dargestellt. Für die D-Amidase ergibt sich daraus ein Temperatur-Optimum im Bereich von 47 und 48°C. Fig. 3 zeigt eine breites pH-Optimum im Bereich von 7,5 bis 9,5 mit etwa gleicher Aktivität. Im NatriumCarbonat-Puffer bei pH 9 wurde die höchste Aktivität ermittelt.

Ein Vergleich der gesamten Aminosäuresequenz der erfindungsgemäßen D-Amidase mit vorhandenen Amidasen in Proteindatenbanken bei NCBI via Internet (http://www.ncbi.nlm.nib.gov/blast mit Programm: BLASTP 2.2.1, Database: nr, pat, SwissProt) führt zu Ähnlichkeiten mit einer Vielzahl an bekannten Amidasen. Die Ähnlichkeit der Aminosäuresequenz beträgt im besten Fall 66 % (303/457 AS) bzw. 50 %. Identität (229/457 AS) mit einer möglichen Amidase aus *Mycobacterium tuberculosis*, (gi: 6225047; Cole, S. T. set al. (1998), Deciphering the biology of Mycobacterium tuberculosis from the complete genome sequence, Nature 393, 537-544). Die enantioselektive Amidase aus *Rhodococcus sp*. ist mit 52% bzw 37 % das zweitähnlichste Protein, (gi: 152052; Mayaux, J.-F.; Cerbelaud; E.; Soubrier, F.; Yeh, P.; Blanche, F.; Pétré, D. (1991), Purification, Cloning, and Primary Structure of a New Enantiomer-Selective Amidase from a Rhodococcus Strain: Structural E-vidence for a Conserved Genetic Coupling with Nitrile Hydatase, J. Bacteriol. 173, 6694-6704) das auch schon beim Vergleich des N-Terminus mit der D-Amidase eine hohe Homologie besitzt.

Bei der vorliegenden Erfindung handelt es sich also um eine neue D-Amidase mit der Fähigkeit u.a. DL-Tle-NH₂ in ausgezeichneten ee-Werten und Ausbeuten in D-Tle zu verwandeln.

Da bisher diese optische Antipode der Aminosäure nur schwer zugänglich war, bedeutet die Angabe der neuen D-Amidasen einen wichtigen Schritt zur technischen Herstellung dieser unnatürlichen Aminosäure, welche vorzugweise in bioaktiven Peptidmimetika Anwendung finden kann.

Für die Anwendung kann das betrachtete Enzym in freier Form als homogen aufgereinigte Verbindungen oder als rekombinant hergestelltes Enzym verwendet werden. Weiterhin kann das Enzym auch als Bestandteil eines intakten Gastorganismus eingesetzt werden oder in Verbindung mit der aufgeschlossen und beliebig hoch aufgereinigten Zellmasse des Wirtsorganismus Möglich ist ebenfalls die Verwendung der Enzyme in immobilisierter Form (Sharma B. P.; Bailey L. F. und Messing R. A. (1982), Immobilisierte Biomaterialiern - Techniken und Anwendungen, Angew. Chem. 94, 836-852). Vorteilhafterweise erfolgt die Immobilisierung durch Lyophilisation (Paradkar, V. M.; Dordick, J. S. (1994), Aqueous-Like Activity of α-Chymotrypsin Dissolved in Nearly Anhydrous Organic Solvents, J. Am. Chem. Soc. 116, 5009-5010; Mori; T.; Okahata, Y. (1997), A variety of lipi-coated glycoside hydrolases as effective glycosyl transfer catalysts in homogeneous organic solvents, Tetrahedron Lett. 38, 1971-1974; Otamiri. M.; Adlercreutz, P.; Matthiasson, B. (1992), Complex formation between chymotrypsin and ethyl cellulose as a means to solbilize the enzyme in active form in toluene, Biocatalysis 6, 291-305). Ganz besonders bevorzugt ist die Lyophilisation in Gegenwart von oberflächenaktiven Substanzen, wie Aerosol OT oder Polyvinylpyrrolidon oder Polyethylenglycol (PEG) oder Brij 52 (Diethylenglycolmono-cetylether) (Kamiya, N.; Okazaki, S.-Y.; Goto, M. (1997), Surfactant-horseradish peroxidase complex catalytically active in anhydrous benzene, Biotechnol. Tech. 11, 375-378). Die Verwendung als, CLECs ist ebenfalls denkbar (St. Clair, N.; Wang, Y.-F.; Margolin, A. L. (2000), Cofactor-bound cross-linked enzyme crystals (CLEC) of alcohol dehydrogenase, Angew. Chem. Int. Ed. 39, 380-383).

Unter optisch angereicherten (enantiomerenangereicherten enantiomer angereicherten) Verbindungen wird im Rahmen der Erfindung das vorliegen einer optischen Antipode im Gemisch mit der andern in >50 mol-% verstanden.

Unter dem Begriff Nukleinsäuren, werden alle Arten von einzelsträngiger oder doppelsträngiger DNA als auch RNA oder Gemische derselben subsumiert.

Unter verbesserten rec-Enzymen werden anspruchsgemäß insbesondere solche - verstanden, die ein modifiziertes Substratspektrum aufweisen, aktiver und/oder selektiver oder unter den verwendeten Reaktionsbedingungen stabiler sind.

Von den beanspruchten Proteinsequenzen und den Nukleinsäuresequenzen werden erfindungsgemäß auch solche Sequenzen umfaßt, die eine Homologie (exclusive der natürlichen Degeneration) größer als 80 %, bevorzugt größer als 90 %, 91 %, 92 %, 93 % oder 94 %, mehr bevorzugt größer als 95 % oder 96 % und besonderes bevorzugt größer als 97 %, 98 % oder 99 % zu einer dieser Sequenzen aufweisen, sofern die Wirkungsweise bzw. Zweck einer solchen Sequenz erhalten bleibt. Der Ausdruck "Homologie" (oder Identität) wie hierin verwendet, kann durch die Gleichung H (%) = [1 - V/X] x 100 definiert werden, worin H Homologie bedeutet, X die Gesamtzahl an Nukleobasen/Aminosäuren der Vergleichssequenz ist und V die Anzahl an unterschiedlichen Nukleobasen/Aminosäuren der zu betrachtenden Sequenz bezogen auf die Vergleiehssequenz ist. Auf jeden Fall sind mit dem Begriff Nukleinsäuren, welche für Aminosauresequenzen codieren, alle Sequenzen umfaßt, die nach Maßgabe der Degeneration des genetischen Codes möglich erscheinen.

Die in dieser, Schrift genannten Literaturstellen gelten als von der Offenbarung mitumfaßt.

### SEQUENZPROTOKOLL

<110> Degussa AG
<120> Amidase aus Variovorax paradoxus
<130> 010199 AM
<140>
   <141>
<160> 13
<170> PatentIn Ver. 2.1
<210> 1
   <211> 1398
   <212> DNA
   <213> Variovorax paradoxus
<220>
   <221> CDS
   <222> (1)..(1398)
<400> 1
   atg agc aac gaa ctg cat tac ctc gag ctg gtc gac gtc ggc cgg cgc    48
   Met Ser Asn Glu Leu His Tyr Leu Glu Leu Val Asp Val Gly Arg Arg
    1               5                  10                  15
   atc cag cgc aag gag ctc tcg ccg gtc gag gtc acg caa gcg cag ctc    96
   Ile Gln Arg Lys Glu Leu Ser Pro Val Glu Val Thr Gln Ala Gln Leu
               20                  25                  30
   gcg cgc atc gag aag gtc gac ggc gcg ctc aag agc tat gtg atc gtg   144
   Ala Arg Ile Glu Lys Val Asp Gly Ala Leu Lys Ser Tyr Val Ile Val
           35                  40                  45
   atg gcc gaa cac gcg ctg gcc gac gcg cgc cgc gcc gag gcc gag atc   192
   Met Ala Glu His Ala Leu Ala Asp Ala Arg Arg Ala Glu Ala Glu Ile
       50                  55                  60
   gcc cgg ggc gag atc cgc ggg ccg ctg cac ggc gtg ccg gtg gcg gtg   240
   Ala Arg Gly Glu Ile Arg Gly Pro Leu His Gly Val Pro Val Ala Val
   65                  70                  75                  80
   aag gac ctg tgc tgg aca aaa ggc gtg gcc acg gcc gcc ggc atg acg   288
   Lys Asp Leu Cys Trp Thr Lys Gly Val Ala Thr Ala Ala Gly Met Thr
                   85                  90                  95
   ctc tac cgc gac ttc gtg ccc acc gag gac ggc acg gcc gtg cgc aag   336
   Leu Tyr Arg Asp Phe Val Pro Thr Glu Asp Gly Thr Ala Val Arg Lys
               100                 105                 110
   ctg cgc gaa gcc ggt gcc gtg atc ctc ggc aag ctg cag ctc acc gag   384
   Leu Arg Glu Ala Gly Ala Val Ile Leu Gly Lys Leu Gln Leu Thr Glu
           115                 120                 125
   agc gcc tat gcc gac cat cac ccc agc gtc acg ccg ccg gtc aat ccg   432
   Ser Ala Tyr Ala Asp His His Pro Ser Val Thr Pro Pro Val Asn Pro
       130                 135                 140
   tgg aac gcg gcg cac tgg tcg ggt gcc tcg tcg agc ggc tcg ggc gtg   480
   Trp Asn Ala Ala His Trp Ser Gly Ala Ser Ser Ser Gly Ser Gly Val
   145                 150                 155                 160
   gcg acc gcg gcg ggg ctt tgc tat ggc tcg ctc ggc acc gac acg ggc   528
   Ala Thr Ala Ala Gly Leu Cys Tyr Gly Ser Leu Gly Thr Asp Thr Gly
                   165                 170                 175
   ggc tcg atc cgc ttt ccg tcc tcg gcc aac ggc ctg acc ggc ctg aag   576
   Gly Ser Ile Arg Phe Pro Ser Ser Ala Asn Gly Leu Thr Gly Leu Lys
               180                 185                 190
   ccg acc tgg ggc cgc gtg agc cgc cat ggc gcc ttc gag ctg gcc gcc   624
   Pro Thr Trp Gly Arg Val Ser Arg His Gly Ala Phe Glu Leu Ala Ala
           195                 200                 205
   acg ctc gac cac atc ggc ccg atg acg cgc agc gcg gcc gat gcg ggt   672
   Thr Leu Asp His Ile Gly Pro Met Thr Arg Ser Ala Ala Asp Ala Gly
       210                 215                 220
   gcg atg ctc ggc gcc atc gcg gga gcc gat ccg aag gac ccg acc gcg   720
   Ala Met Leu Gly Ala Ile Ala Gly Ala Asp Pro Lys Asp Pro Thr Ala
   225                 230                 235                 240
   agc ctc gcg gcc gtg ccc aac tac ctc gcg ggc atg gag cgc ggc ttg   768
   Ser Leu Ala Ala Val Pro Asn Tyr Leu Ala Gly Met Glu Arg Gly Leu
                   245                 250                 255
   cgc ggc ctg cgc gtg ggc atc gac gcg cgc tgg aac gcg gag ggc gtc   816
   Arg Gly Leu Arg Val Gly Ile Asp Ala Arg Trp Asn Ala Glu Gly Val
               260                 265                 270
   gat gcg gcc acc gcg cag gtg atg gaa ggc gcg ctc gcg gcc gtg cgc   864
   Asp Ala Ala Thr Ala Gln Val Met Glu Gly Ala Leu Ala Ala Val Arg
           275                 280                 285
   gaa ctc ggc gcc gaa gtg cgt cac gtg aca ttc ccc gac ccg gcg cag   912
   Glu Leu Gly Ala Glu Val Arg His Val Thr Phe Pro Asp Pro Ala Gln
       290                 295                 300
   gtc atc gcc gac tgg ttc ccg ctg tgc ggc atc gag gcg gcc gtg gtg   960
   Val Ile Ala Asp Trp Phe Pro Leu Cys Gly Ile Glu Ala Ala Val Val
   305                 310                 315                 320
   cac gag tcg acc tat ccc gcg cgc aag cag atg tac ggc ccg gcg ctg  1008
   His Glu Ser Thr Tyr Pro Ala Arg Lys Gln Met Tyr Gly Pro Ala Leu
                   325                 330                 335
   tcg ggc ctg ctc gag ctg ggc cgt gcg caa agc ggc atc gac tac cag  1056
   Ser Gly Leu Leu Glu Leu Gly Arg Ala Gln Ser Gly Ile Asp Tyr Gln
               340                 345                 350
   aag atc gtg ctc cgc cgc cat gcc ttc agc ggc agg gtg cgc gag atg  1104
   Lys Ile Val Leu Arg Arg His Ala Phe Ser Gly Arg Val Arg Glu Met
           355                 360                 365
   ttc gag ggc atc gac ctg ctg ctg atg ccc tcg cag ggc gtc gcc tcg  1152
   Phe Glu Gly Ile Asp Leu Leu Leu Met Pro Ser Gln Gly Val Ala Ser
       370                 375                 380
   ccc acg ctc gcg cgc atg ctg agc ttc ggc gag gac gcc gaa ctg atg     1200
   Pro Thr Leu Ala Arg Met Leu Ser Phe Gly Glu Asp Ala Glu Leu Met
   385                 390                 395                 400
   tcc gcg atg ctg cgc tac acc tgc ccg ctg gac atg agc ggc agc ccg     1248
   Ser Ala Met Leu Arg Tyr Thr Cys Pro Leu Asp Met Ser Gly Ser Pro
                   405                 410                 415
   acg atc acg ctg ccc ggc ggc ttc acc gat gcg ggc acg ccg gtc gca     1296
   Thr Ile Thr Leu Pro Gly Gly Phe Thr Asp Ala Gly Thr Pro Val Ala
               420                 425                 430
   ttc cag ttc gtc gcg cgc cac ttc gag gaa gag ctg ctg gtg cgc gcc     1344
   Phe Gln Phe Val Ala Arg His Phe Glu Glu Glu Leu Leu Val Arg Ala
           435                 440                 445
   ggc tgg gcc ttc cag cag gcg acc gac tgg cac cgg cgg cat ccg gtg     1392
   Gly Trp Ala Phe Gln Gln Ala Thr Asp Trp His Arg Arg His Pro Val
       450                 455                 460
   ctg tag                                                             1398
   Leu
   465
<210> 2
   <211> 465
   <212> PRT
   <213> Variovorax paradoxus
<400> 2
   Met Ser Asn Glu Leu His Tyr Leu Glu Leu Val Asp Val Gly Arg Arg
   1               5                   10                  15
   Ile Gln Arg Lys Glu Leu Ser Pro Val Glu Val Thr Gln Ala Gln Leu
               20                  25                  30
   Ala Arg Ile Glu Lys Val Asp Gly Ala Leu Lys Ser Tyr Val Ile Val
           35                  40                  45
   Met Ala Glu His Ala Leu Ala Asp Ala Arg Arg Ala Glu Ala Glu Ile
       50                  55                  60
   Ala Arg Gly Glu Ile Arg Gly Pro Leu His Gly Val Pro Val Ala Val
   65                  70                  75                  80
   Lys Asp Leu Cys Trp Thr Lys Gly Val Ala Thr Ala Ala Gly Met Thr
                   85                  90                  95
   Leu Tyr Arg Asp Phe Val Pro Thr Glu Asp Gly Thr Ala Val Arg Lys
               100                 105                 110
   Leu Arg Glu Ala Gly Ala Val Ile Leu Gly Lys Leu Gln Leu Thr Glu
           115                 120                 125
   Ser Ala Tyr Ala Asp His His Pro Ser Val Thr Pro Pro Val Asn Pro
       130                 135                 140
   Trp Asn Ala Ala His Trp Ser Gly Ala Ser Ser Ser Gly Ser Gly Val
   145                 150                 155                 160
   Ala Thr Ala Ala Gly Leu Cys Tyr Gly Ser Leu Gly Thr Asp Thr Gly
                   165                 170                 175
   Gly Ser Ile Arg Phe Pro Ser Ser Ala Asn Gly Leu Thr Gly Leu Lys
               180                 185                 190
   Pro Thr Trp Gly Arg Val Ser Arg His Gly Ala Phe Glu Leu Ala Ala
           195                 200                 205
   Thr Leu Asp His Ile Gly Pro Met Thr Arg Ser Ala Ala Asp Ala Gly
       210                 215                 220
   Ala Met Leu Gly Ala Ile Ala Gly Ala Asp Pro Lys Asp Pro Thr Ala
   225                 230                 235                 240
   Ser Leu Ala Ala Val Pro Asn Tyr Leu Ala Gly Met Glu Arg Gly Leu
                   245                 250                 255
   Arg Gly Leu Arg Val Gly Ile Asp Ala Arg Trp Asn Ala Glu Gly Val
               260                 265                 270
   Asp Ala Ala Thr Ala Gln Val Met Glu Gly Ala Leu Ala Ala Val Arg
           275                 280                 285
   Glu Leu Gly Ala Glu Val Arg His Val Thr Phe Pro Asp Pro Ala Gln
       290                 295                 300
   Val Ile Ala Asp Trp Phe Pro Leu Cys Gly Ile Glu Ala Ala Val Val
   305                 310                 315                 320
   His Glu Ser Thr Tyr Pro Ala Arg Lys Gln Met Tyr Gly Pro Ala Leu
                   325                 330                 335
   Ser Gly Leu Leu Glu Leu Gly Arg Ala Gln Ser Gly Ile Asp Tyr Gln
               340                 345                 350
   Lys Ile Val Leu Arg Arg His Ala Phe Ser Gly Arg Val Arg Glu Met
           355                 360                 365
   Phe Glu Gly Ile Asp Leu Leu Leu Met Pro Ser Gln Gly Val Ala Ser
       370                 375                 380
   Pro Thr Leu Ala Arg Met Leu Ser Phe Gly Glu Asp Ala Glu Leu Met
   385                 390                 395                 400
   Ser Ala Met Leu Arg Tyr Thr Cys Pro Leu Asp Met Ser Gly Ser Pro
                   405                 410                 415
   Thr Ile Thr Leu Pro Gly Gly Phe Thr Asp Ala Gly Thr Pro Val Ala
               420                 425                 430
   Phe Gln Phe Val Ala Arg His Phe Glu Glu Glu Leu Leu Val Arg Ala
           435                 440                 445
   Gly Trp Ala Phe Gln Gln Ala Thr Asp Trp His Arg Arg His Pro Val
       450                 455                 460
   Leu
   465
<210> 3
   <211> 29
   <212> DNA
   <213> Künstliche Sequenz
<220>
   <223> Beschreibung der künstlichen Sequenz:Primer AAH-N1
<400> 3
   gtaggccgac gaatccagca gaagga 26
<210> 4
   <211> 29
   <212> DNA
   <213> Künstliche Sequenz
<220>.
   <223> Beschreibung der künstlichen Sequenz:Primer AAH-C1
<400> 4
   gggatacgga tcgagccgcc agtatc 26
<210> 5
   <211> 509
   <212> DNA
   <213> Künstliche Sequenz
<220>
   <223> Beschreibung der künstlichen Sequenz:Gensonde
<400> 5
   gttggccggc gcatccagcg caaggagctc tcgccggtcg aggtcacgca agcgcagctc 60
   gcgcgcatcg agaaggtcga cggcgcgctc aagagctatg tgatcgtgat ggccgaacac 120
   gcgctggccg acgcgcgccg cgccgaggcc gagatcgccc ggggcgagat ccgcgggccg 180
   ctgcacggcg tgccggtggc ggtgaaggac ctgtgctgga caaaaggcgt ggccacggcc 240
   gccggcatga cgctctaccg cgacttcgtg cccaccgagg acggcacggc cgtgcgcaag 300
   ctgcgcgaag ccggtgccgt gatcctcggc aagctgcagc tcaccgagag cgcctatgcc 360
   gaccatcacc ccagcgtcac gccgccggtc aatccgtgga acgcggcgca ctggtcgggt 420
   gcctcgtcga gcggctcggg cgtggcgacc gcggcggggc tttgctatgg ctcgctcggc 480
   accgacacgg gcggctcgat ccgctttcc                                  509
<210> 6
   <211> 28
   <212> DNA
   <213> Künstliche Sequenz
<220>
   <223> Beschreibung der künstlichen Sequenz:Primer AAH-GW-F1
<400> 6
   gcgtcacgcc gccggtcaat ccgtggaa 28
<210> 7
   <211> 26
   <212> DNA
   <213> Künstliche Sequenz
<220>
   <223> Beschreibung der künstlichen Sequenz:Primer AAH-GW-P2
<400> 7
   ggcgctgg tcgggtgcct cgtcga 26
<210> 8
   <211> 30
   <212> DNA
   <213> Künstliche Sequenz
<220>
   <223> Beschreibung der künstlichen Sequenz:Primer AAH-GW-R1
<400> 8
   cagcgcgtgt tcggccatca cgatcacata 30
<210> 9
   <211> 28
   <212> DNA
   <213> Künstliche Sequenz
<220>
   <223> Beschreibung der künstlichen Sequenz:Primer AAH-GW-R2
<400> 9
   ctcttgagcg cgccgtcgac cttctcga 28
<210> 10
   <211> 27
   <212> DNA
   <213> Künstliche Sequenz
<220>
   <223> Beschreibung der künstlichen Sequenz:Primer AAH-K-N2
<400> 10
   ctggtcatca agcgcggcca gatcggc 27
<210> 11
   <211> 26
   <212> DNA
   <213> Künstliche Sequenz
<220>
   <223> Beschreibung der künstlichen Sequenz:Primer AAH-K-C2
<400> 11
   gatcggccga cagccgattg gccagc 26
<210> 12
   <211> 32
   <212> DNA
   <213> Künstliche Sequenz
<220>
   <223> Beschreibung der künstlichen Sequenz:Primer AAH-N-EcoRI
<400> 12
   ccggaattca tgagcaacga actgcattac ct 32
<210> 13
   <211> 29
   <212> DNA
   <213> Künstliche Sequenz
<220>
   <223> Beschreibung der künstlichen Sequenz:Primer AAH-C-HindIII
<400> 13
   atcccaagct tttacagcac cggatgccg 29

### Beispiele:

### 1. Anzucht des Stamms Variovorax paradoxus DSM 14468 zur Induktion der D-Amidase

Der Stamm *Variovorax paradoxus* 19-3 DSM 14468 wurde zur Induktion der D-Amidase in einem Minimalmedium mit racemischem Tle-NR₂ als Stickstoffquelle kultiviert; Zusammensetzung des Minimalmediums:

**Tabelle 6: Minimalmedium für Variovorax paradoxus**

| Minimalmedium | |
|---|---|
| KH₂PO₄ | 3,30g |
| K₂HPO₄ | 0,80 g |
| NaCl | 1,00 g |
| CaCl₂ | 0,05 g |
| MgSO₄ x 7 H₂O | 0,30 g |
| D,L-tertiär-Leucinamid | 3,30 g |
| Glucose | 4,50 g |
| Spurensalzlösung | 0,80 ml |
| Vitaminlösung | 2,50 ml |
| Aq. demin. | ad 1000 ml; pH 7,3 |

**Tabelle 7: Zusaamensetzung der Spurensalzlösung**

| Spurensalzlösung | |
|---|---|
| H₃BO₃ | 75,0 mg |
| MnCl₂ x 4 H₂O | 50,0 mg |
| ZnCl₂, | 187,5 mg |
| CuSO₄ x 5 H₂O | 50,0 mg |
| FeCl₃ | 625,0 mg |
| (NH₄)₆Mo₇O₂₄ x 4 H₂O | 25,0 mg |
| CoCl₂ x 6 H₂O | 37,5 mg |
| NiCl₂ x 6 H₂O | 50,0 mg |
| Aq. demin. | ad 200 ml |

**Tabelle 8: Zusammensetzung der Vitaminlösung**

| Vitaminlösung | |
|---|---|
| Biotin | 0,20 mg |
| Nicotinsäure | 2,00 mg |
| Thiamin | 1,00 mg |
| 4-Aminobenzoat | 1,00 mg |
| Panthothenat | 0,50 mg |
| Pyridoxamin | 5,00 mg |
| Cyanocobalamin | 2,00 mg |
| Aq. demin. | ad 100 ml |

Die Sterilisation erfolgte durch 20-minütiges Autoklavieren bei 121 °C und 1,2 bar. Da Glucose, CaCl₂, MgSO₄ x 7 H₂O, Vitaminlösung, DL-Tle-NH₂ auf diese Art der Sterilisation empfindlich reagieren, wurden sie den Nährlösungen erst nach dem Autoklavieren durch Sterilfiltration über 0,2 µm Membranen (Sartorius) hinzugefügt.

### 2. Gewinnung von Rohextrakten aus Variovorax paradoxus und E. coli

Die Kulturen wurde nach der Ernte durch Zentrifugation mit Kaliumphosphat-Puffer, 100. mM pH 7,5 einmal gewaschen rund eine 20%ige Zellsuspension eingestellt. Der Zellaufschluß im analytischen Maßstab erfolgte entweder durch Naßvermahlung mittels einer Schwingmühle der Firma Retsch (Hummel, W.; Kula, M.-R. (1989). A Simple Method for Small-Scale Disruption of Bacteria and Yeasts, J. Microbiol. Methods 9, 201-209), oder durch Ultraschall mittels eines Pulse Sonifier der Firma Branson.

Zur Erniedrigung von Protease-Aktivitäten erfolgten alle weiteren Arbeitsschritte unter Kühlung auf 4°C. Die Proteingehaltsbestimmung mittes Bradford (Bradford, M. M. (1976), A Rapid and Sensitive Methode for the Quantitation of Microgram Quantities of Protein Utilizing the Principle of Protein-Dye Binding, Anal. Biochem. 72, 248-254) im Rohextrakt ermöglichte die Beurteilung der Aufschlußgüte.

### Naßvermahlung mittels Schwingmühle:

In 1,5 ml Eppendorf-Cups wurden 1,2 g Glasperlen (Durchmesser 0,3 mm) und 0,6 ml Zellsuspension gefüllt und mittles einer Schwingmühle zehn min bei maximaler Schwingfrequenz ausgeschlossen. Aus dem Zellhomogenat wurden Glasperlen und Zelltrümer mittels zehnminütiger Zentrifugation bei 10.000 rpm und 4°C abgetrennt und der Überstand als Rohextrakt in den Enzymtests eingesetzt.

### Ultraschallaufschluß:

*Variovorax paradoxus Kulturen* wurden in Portionen von maximal 10 ml mit 8 mal 60 s-bursts bei 70 %Puls, 80 %Intensität und jeweiligem 60 s Zwischenkühlen aufgeschlossen. *E. coli* Kulturen wurden in 1 ml Portionen mit 4 mal 60 s-bursts bei 70 % Puls, 80 %Intensität und jeweiligem 60 s Zwischenkühlen aufgeschlossen. Das Zellhomogenat wurde abzentrifugiert und der Rohextrakt abgenommen.

Aufschluß zur Aufreinigung der D-Amidase aus *Variovorax paradoxus* von Volumina zwischen 20 und 200 ml wurde in einem Desintegrator S der Firma IMA durchgeführt. Dazu wurden Zellsuspension und Glasperlen (Durchmesser 0,3 mm) in einem Verhältnis 1:1,5 gemischt und die Zellen 20 min bei 3500 rpm aufgeschlossen.

### 2. Anzucht das Stamms Variovorax paradoxus DSM 14468 im DSMZ-Vollmedium Nr.1 und Präparation der genomischen DNA

Hierzu wurde der Stamm *Variovorax paradoxus* im DSMZ-Vollmedium Nr.1 bis zu einer optischen Dichte OD₆₆₀ von ca. 1,0 kultiviert; Zusammensetzung (DSMZ, Catalogue of Strains (1938), Braunschweig):

**Tabelle 9: Zusammensetzung des DSMZ-Vollmediums Nr. 1**

| DSMZ-Vollmedium Nr.1 | |
|---|---|
| Pepton | 5,0 g |
| Fleischextrakt | 3,0 g |
| Aq. demin. | ad 1000 ml; pH 7,0 |

Die Kultur wurde unter sterilen Bedingungen geerntet und einmal mit sterilem Kaliumphosphat-Puffer 20 mM pH 6,5 gewaschen.

Die Präparation der genomischen DNA erfolgte mittels Dneasy Tissue Kit (Qiagen). Die Durchführung erfolgte gemäß dem Protokoll für gram-negative Bakterien aus dem Qiagen DNeasy Tissue Kit Handbook (4/99).

### 3. Oligonucleotide

**Tabelle 10: Liste der verwendeten Oligonucleotide**

| Bezeichnung : | Verwendung : | sequenz : |
|---|---|---|
| AAH-N1 | PCR | |
| AAH-C1 | PCR | |
| AAH-GW-F1 | PCR | |
| AAH-GW-R1 | PCR | |
| AAH-GW-F2 | PCR | |
| AAH-GW-R2 | PCR | |
| AAH-K-N2 | PCR | |
| AAH-K-C2 | PCR | |
| AAH-N-EcoRI | PCR | |
| AAH-C-HindIII | PCR | |

Die Bezeichnung S steht für G + C in der Sequenz von AAH-N1 und AAH-C1 (IUB Gruppencode zur Identifizerung von Redundanzen).

### 4. Gentechnische Methoden

Alle hier verwendeten gentechnischen Methoden sind, wenn nicht anders vermerk, beschrieben von Sambrook et al. (Sambrook, J.; Fritsch, E. F. und Maniatis, T. (1989), Molecular cloning: a laboratory manual, 2nd ed., Cold Spring Harbor Laboratory Press, New York) und Hopwood et al. (Hopwood, D. A.; Bibb, M. J.; Chater, K. F.; Kieser, T.; Bruton, C. J.; Kieser, H. M.; Lydiate, D. J.; Smith, C. P.; Ward, J. M.; Schrempf, H. (1985), Genetic manipulation of Stretomycetes: A laboratory manual, The John Innes Foundation, Norwich). Alle Enzyme und entsprechende Puffer wurden nach Angaben der Hersteller benutzt. Die Sequenzierungen erfolgte durch die Firma Sequiserve, Vaterstetten.

### 5. Polymerasekettenreaktion (PCR)

DNA-Amplifikationen mittels der Polymerasekettenreaktion wurde mit dem Biometra Personal Cycler^{™} (Göttingen) in Anlehnung an Saiki et al. (Saiki, R. K.; Gelfand, D. H.; Stoffel, S.; Scharf, S. J.; Higuchi, R.; Horn, G. T.; Mullis, R. B.; Ehrlich, H. A. (1988), Primer-directed enzymatic amplification of DNA with a thermostable DNA polymerase, Science 239; 487-491) durchgeführt.

Es wurden die thermostabilen DNA-Polymerasen Vent_{R}®. (New England Biolabs) und Herculase^{™} (Stratagene) und die vom Hersteller mitgelieferten Puffer verwendet. Die verwendeten Primerpärchen sind in der Tabelle 10 aufgelistet.

Durch PCR wurde zunächst ein 509 bp großes Fragment des D-Amidase-Gens, nach Vorliegen der gesammten Gensequenz der D-Amidase durch die GenomeWalker Methode (Stratagene) das gesammte D-Amidase-Gen aus genomischer DNA amplifizert. Nach Sicherung und Überprüfung dieser D-Amidase Gensequenz in dem Vektor pUC18 wurde Plasmid-DNA als Template benützt.

**Tabelle 11: Zusammensetzung eines PCR-Ansatzes für genomische oder Plasmid-DNA**

| PCR-Ansatz: | |
|---|---|
| genomische DNA oder | 100 - 500 ng |
| Plasmid-DNA | 20 ng |
| Polymerase-Puffer (10x). | 1/10 Vol. |
| dNTP's | je 0,2 mM |
| Sense- und Antisense-Primer | je 10 - 50 pmol |
| DNA-Polymerase | 1 - 5 U |
| DMSO | 5 % (v/v) |
| Aq. demin. | ad 50 µl |

Die PCR-Ansätze wuren mit ca. 50 µl leichtem Mineralöl überschichtet. Für die PCR-Programme wurde die Annealing-Temperatur TA über die DNA-Schmelztemperatur (Tm) der Oligonucleotide ermittelt- Die Zeit X für die Kette-reaktion der DNA-Polymerase richtete sich nach der 1 kb = 1 min-Regel.

**Tabelle 12: PCR-Progrmm für 0,5 kb Fragment des D-Amidase-Gens (Seq. 5) mit Primerpaar AAH-N1/AAH-C1**

| Schritt | Temperatur | Zeit |
|---|---|---|
| 1 | 95°C | 5 min |
| 2 | 95°C | 1 min |
| 3 | 70 - 61°C | 45 sec |
| 4 | 72°C | 30 sec |
| 5 | 95°C | 1 min |
| 6 | 70°C | 45 sec |
| 7 | 72°C | 30 sec |
| 8 | 72°C | 2 min |

Die Schritte 2 - 4 wurde 10 mal durchlaufen, wobei die Annealingtemperatur von Schritt 3, um -1°C pro Cyclus erniedrigt wurde. Anschließend wurden die Schritte 5 - 7 20 mal durchlaufen.

**Tabelle 13: PCR-Programm für gesamtes D-Amidase-Gen (Seq. 1) mit 92 bp vor Startcodon und 80 bp nach dem Stopcodon mit Primerpaar AAH-K-N2/AAH-K-C2**

| schritt | Temperatur | Zeit |
|---|---|---|
| 1 | 95°C | 5 min |
| 2 | 95°C | 45 sec |
| 3 | 70°C | 2,5 min |
| 4 | 95°C | 45 sec |
| 5 | 67°C | 2,5 min |
| 6 | 67°C | 5 min |

Die Schritte 2 - 3 wurden 10 mal und anschließend die Schritte 4 - 520 mal durchlaufen.

**Tabelle 14: PCR-Programm, für die Einführung einer EcoRI- am 5'-Ende und eine HindIII-Schnittstelle am 3'-Ende des D-Amidase-Gens mit Primerpaa AAH-N-EcorI/AAH-C-HinIII:**

| Schritt, | Temperatur | Zeit |
|---|---|---|
| 1 | 95°C | 5 mit |
| 2 | 95°C | 1 min |
| 3 | 52 - 41°C | 1min |
| 4 | 70°C | 2 min |
| 5 | 95°C | 1 min |
| 6 | 62°C | 1 min |
| 7 | 70°C | 2 min |
| 8 | 70°C | 5 min |

Die Schritte 2-4 wurden 12 mal durchlaufen, wobei die Annealingtemperatur von Schritt 3, um -1°C pro Cyclus erniedrigt wurde. Anschließend wurden die Schritte 5 - 7 20 mal durchlaufen.

Die Reinigung und Isolierung der PCR-Produkte erfolgte entweder direkt mittels des PCR Purification Kits (Qiagen) oder durch Agarosegelelektrophorese des gesammten PCR-Ansatzes und anschließende Isolierung des DNA-Fragmentes mittels QIAquick Gel Extraktion Kit (Qiagen).

### 6. Universal GenomeWalker^{™}-Methode (Clontech) zur Auffindung das gesamten D-Amidase-Gen.

Anhand des bereits bekannten 509 bp große Fragment des D-Amidase-Gens (Seq. 5) ermöglichte das Universal GenomeWalker^{™} Kit ausgehend von genomischer DNA aus *Variovorax paradoxus* eine PCR-vermittelte Klonierung des gesamten D-Amidasegens.

Für eine sogenannte "GenomeWalker Bibliothek" wurde genomische DNA mit vier verschiedenen Restriktionsenzymen (*EcoR V, Sca I*, *Pvu II* und *Stu I*) hydolysiert. Nach Aufreinigung der DNA-Restriktionsansätze folgte jeweils eine blunt-end Ligation mit den beigefügten GenomeWalker Adaptoren, für die zwei Adaptor-Primer (AP1 und AP2) vorlagen. Die genomische DNA-Restriktion sowie die Ligation der Adaptoren erfolgte gemäß dem Universal GenomeWalker^{™} Kit User Manual (3/2000, Clontech).

Diese Bibliothek diente als Template für eine erste PCR-Reaktion (Primary PCR). Für je eine PCR-Reaktion in beiden Richtungen wurden die Primerpaare AP1/AAH-GW-F1 (downstream) und AP1/AAH-GW-R1 (upstream) verwendet. Um eine Amplifizerung von großen DNA-Fragmenten zu ermöglichen ("long-distance PCR") wurde die Herculase® (Stratagene) als DNA-Polymerase eingesetzt.

**Tabelle 15: Zusammensetzung: 1.PCR, GenomeWalker^{™}-Methode**

| PCR-Ansatz : | |
|---|---|
| GenomeWalker-Bibliothek | 2 µl |
| Polymerase-Puffer (10x) | 1/10 Vol. |
| dNTP's | je 0,2 mM |
| Sense- und Antisense-Primer | 50 pmol |
| DNA-Polymerase | 2,5 U |
| DMSO | 5 % (v/v) |
| Aq. demin. | ad 50 µl |

Die PCR-Ansätze wuren mit ca. 50 µl leichtem Mineralöl- überschichtet.

**Tabelle 16 : PCR-Programm für 1.PCR, GenomeWalker^{™}-Methode mit Primerpaar AP1/AAH-GW-F1 oder AP1/AAH-GW-R1**

| Schritt | Temperatur | Zeit |
|---|---|---|
| 1 | 95°C | 2 min |
| 2 | 95°C | 30 sec |
| 3 | 70°C | 4min |
| 4 | 95°C | 30 sec |
| 5 | 67°C | 4 min |
| 6 | 67°C | 10min |

Die Schritte 2 - 3 wurden 10 mal durchlaufen. Anschließend wurden die Schritte 4 - 5 25 mal durchlaufen, wobei die Dauer von Schritt 5, um 10 sec pro Cyclus verlängert, wurde (Zeitinkrement).

Mit den erhaltenen PCR-Produkten erfolgte eine zweite PCR (Secondary or nested PCR) mit den Primerpaaren AP2/AAH-GW-F2 (downstream) und AP2/AAH-GW-R2 (upstream), durch die eine Bildung von unspezifischen PCR-Produkten minimiert werden kann. Die PCR-Amplifikate wurden je nach Konzentra-tion bis zu 1:50 mit Aq. demin verdünnt und so als Template eingesetzt.

**Tabelle 17: Zusammensetzung 2. PCR, GenomeWalker^{™}-Methode**

| PCR-Ansatz: | |
|---|---|
| PCR-Produkt von 1.PCR | 1 µl |
| Polymerase-Puffer (10x) | 1/10 Vol. |
| dNTP's | je 0.2 mM |
| Sense- und Antisense-Primer | 50 pmol |
| DNA-Polymerase | 2,5 U |
| DMSO | 5 % (v/v) |
| Aq. demin. | ad 50 µl |

Die PCR-Ansätze wuren mit ca. 50 µl leichtem Mineralöl überschichtet.

**Tabelle 18: PCR-Programm für 2.PCR, GenomeWalker^{™}-Methode mit Primerpaar AP2/AAH-GW-F2 oder AP2/AAH-GW-R2**

| Schritt | Temperatur | Zeit |
|---|---|---|
| 1 | 95°C, | 2 min |
| 2 | 95°C | 30 sec |
| 3 | 70°C | 4 min |
| 4 | 95°C | 30 sec |
| 5 | 67°C | 4 min |
| 6 | 67°C | 10 min |

Die schritte 2 - 3 wurden 10 mal und anschließend die Schritte 4 - 5 20 mal durchlaufen.

Die resultierende PCR-Produkte der 2.PCR wurden in einem Agarosegel getrennt und das überwiegende Amplifikat wie beschrieben aufgereinigt und sequenziert. Zusammenfassend ergaben sich somit ausgehend von der GenomeWalker Bibliothek nach der 2.PCR folgende pCR-Amplifikate:

**Tabelle 19: PCR-Produkte nach 2.PCR, GenomeWalker^{™}-Methode**

| Richtung Downstream | |
|---|---|
| GenomeWalker Bibliothek | Größe 2. PCR-Produkt |
| *EcoR V*-Bibliothek | 1,6 kb |
| *Pvu II*-Bibliothek | 3,5 kb |
| *Sca I*-Bibliothek | 1,2 kb |
| *Stu I*-Bibliothek | 1,4 kb |

| Richtung Upstreamm | |
|---|---|
| GenomeWalker Bibliothek | Größe 2.PCR-Produkt. |
| *EcoR V*-Bibliothek | kein Amplifikat |
| *Pvu II*-Bibliothek | 0,5 kb |
| *Sca I*-Bibliothek | kein Amplifikat |
| *Stu I*-Bibliothek | 1,8 kb |

### 6. Ligation der PCR-Produkte in den Vektor pUC18 und anschließende Klonierung in E. coli XL1 Blue

Die PCR-Produkte aus drei parallelen Ansätzen mit dem gesamten D-Amidase-Gen mit 92 bp vor Startcodon und 80 bp nach dem Stopcodon wurden für die weiteren Arbeiten in den Vektor pUC18 (Roche Biochemicals) ligiert und in *E*. *coli* XL1 Blue transformiert. Diese Techniken sind ausführlich in Sambrook et al. (Sambrook, J.; Fritsch, E. F. und Maniatis, T. (1989), Molecular cloning: a laboratory manual, 2nd ed." Cold Spring Harbor Laboratory Press, New York) und Hopwood et al. (Hopwood, D. A.; Bibb, M. J.; Chater, K. F.; Dieser, T.; Bruton, C. J.; Kieser, H. M.; Lydiate, D. J.; Smith, C. P.; Ward, J. M.; Schrempf, H. (1985), Genetic manipulation of Stretomycetes: A laboratory manual, The John Innes Foundation, Norwich) beschrieben und dem Fachmann daher bekannt.

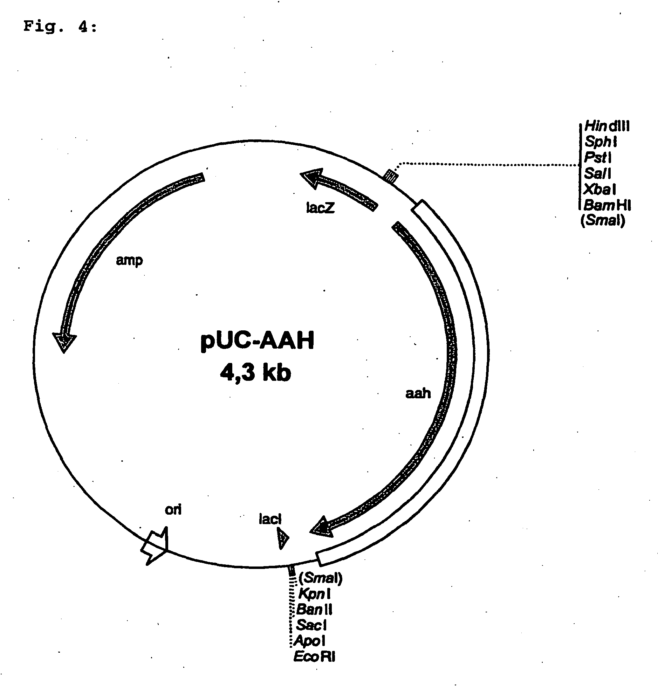

Die D-Amidase-Gensequenz (seq. 1) der Plasmide wurden durch Sequenzierung überprüft und war für die drei Ansätze identisch. Das Plasmid würde pUC-AAH (Fig. 4) genannt. In Fig. 4 ist eine Plasmidkarte von pUC-AAH dargestellt. Gezeigt ist das 1,6 kb große PCR-Produkt mit dem aah Gen und 92 bp vor Startcodon und 80 bp nach dem Stopcodon in pUC18. (Primerpaar : AAH-K-N2/AAH-R-C2)

### 7. Heterologe Expression des D-Amidase-Enzyms aus Variovorax paradoxus in E. coli JM 101

Wie bereits unter 5. Beschrieben wurde ausgehend vom Plasmid pUC-AAH für eine Expression der D-Amidase mittels PCR-Reaktion eine EcoRI- am 5'-Ende und eine HindIII-Schnittstelle am 3'-Ende eingeführt und das PCR-Produkt in den pBTAC-Vektor ligiert und in den Expressionsstamm *E*. *coli* JM 101 transformiert.

Die standardisierte heterologe Expression erfolgte in Anlehnung an Sambrook et al. (Sambrook, J.; Fritsch, E. F. und Maniatis, T. (1989), Molekular cloning: a laboratory manual, 2nd ed., Cold Spring Harbor Laboratory Presse, New York).

Die transformierten *E*. *coli* JM 101-Derivate wurden in LB-Medium (mit 0,1 mg/ml Ampicillin) über Nacht bei 37°C inkubiert. Es wurden dann 30 ml Kulturen (LB-Medium mit 0,1 mg/ml Ampicillin in 4 Schikane-Kolben) mit 0,3 ml Übernachtkultur (1:100) beimpft. Die Expression der Amidase wurde bei einer Zelldichte von OD₆₀₀ₙₘ = 0,4 - 0,6 mit 30 µl einer 1 M IPTG-Lösung (Konzentration in der Kultur 1 mM; IPTG = Isopropylthiogalactosid) induziert. Nach weiteren 4 - 6 h Inkubation bei 30°C wurden die Zellen geerntet.

In Rohextrakten der Expressionsklone, die in oben beschriebener Weise angezogen wurden, konnte eine D-Amidase-Aktivität von 80 - 210 mU/mg Gesamtprotein für DL-Tle-NH₂ als Substrat ermittelt werden.

### 8. Nachweis der D-Amidaseaktivität

Bei der Umsetzung von tertiär-Leucinamid (allg. Säureamiden) durch die D-Amidase kommt es zur Bildung von Ammoniak bzw. Ammoniumionen und tertiär-Leucin (allg. Säure) in äquimolaren Mengen. Als Nachweis der Amidase-Aktivität wurde somit die Bestimmung der Ammoniumionen-Zunahme bzw. die tertiär-Leucin-Bildung mittes HPLC herangezogen. Die Konzentratonsänderung der beteiligten Komponenten kann in einem Enzymtest gemessen werden, der aus, einer 10 - 60 minütigen Inkubation bei 30°C des folgenden Testansatzes besteht.

**Tabelle 20: Zusammensetzung des Enzymtests.**

| Enzymtest | Volumen [µl] |
|---|---|
| Kaliumphosphat-Puffer, (0.1 M, pH 7,5) | 400 |
| DL-tertiär-Leucinamid (0,2 M) | 50 |
| Rohextrakt oder aufgereinigte Enzymlösung. | 50 |
| Gesamtvolumen | 500 |

Durch Zugabe des Substrates wurde der Enzymtest gestartet, der Reaktionsstop erfolgte durch dreiminütiges Erhitzen auf 95°C. Die Analyse der Reaktionsprodukte Ammoniumionen erfolgte durch enzymatische Ammonium-Bestimmung mittels der Glutamat-Dehydrogenase (Bergmeyer, H., U., and Beutler, H.-O. (1985) Ammonia. In: Methods of Enzymatic Analysis. VCH-Verlag, 3rd Edition, Vol. 8: 454-461, Weinheim) bzw. D- und L-tertiär-Leucin mittels HPLC (Brückner, H., Wittner R., and Godel H., (1991) Fully automated high-performance liquid chromatographic separation of DL-amino acids derivatized with o-Phthaldialdehyde together with N-isopropylcysteine. Application to food samples. Anal. Biochem. 144(1): 204-206). Als Kontrollen dienten Ansätze, dienen kein Substrat zugesetzt war.

### 9. Enzymatische Bestimmung der Ammoniumionen mittels der Glutamat-Dehydrogenase

Das Enzym Glutamat-Dehydrogenase (GluDH; E.C.1.4.1.3) setzt 2-Oxoglutarat zu L-Glutamat um, wobei Ammoniumionen verbraucht und NADH zu NAD+ oxidiert werden (Bergmeyer, H., U. und Beutler, H.-O. (1985), Ammonia. In: Methods of Enzymatic Analysis. VCH-Verlag, 3rd Edition, Vol. 8: 454-461, Weinheim).

Die während der Reaktion verbrauchte NADH-Menge ist der Menge an Ammoniumionen äquivalent. Die Konzentrationsänderung an NADH ist die Meßgöße und kann spektrophotometrisch bei einer Wellenlänge von 340 nm bestimmt werden.

### Testlösungen:

2-Oxoglutarat/ADP/TEA-Puffer: 9,3 g TEA, 95 mg ADP, 670 mg
   2-Oxoglutarat in Aq. demin. ad 100 ml, pH 8,0
NADH-Lösung: 30 mg NADH, 60 mg NaHCO3 in 6 ml Aq.demin. gelöst
Glutamat-Dehydrogenase: Aus Rinderleber in 50% Glycerin, 120 U/mg

**Tabelle 21: Zusammensetzung der Ammoniumionen-Bestimmung mittels der Glutamat-Dehydrogenase**

| Testablauf | Volumen [µl] |
|---|---|
| 2-Oxoglutarat/ADP/TEA-Puffer | 500 |
| NADH-Lösung | 50 |
| Probe-Lösung | 100 |
| Aq. demin. | 950 |
| Gesamtvolumen | 1600 |

Die Testkomponenten wurden in Plastikküvetten (1,5 ml halbmikro Einmalküvetten, Brand) pipettiert, vermischt und nach 5 min die Extinktion bei 340 nm bestimmt. Anschließend erfolgte die Zugabe von 10 µl GluDH und nach vollständigem Ablauf der Reaktion, in der Regel nach 30 min, erneut eine Extinktionsmessung.

Die Extinktionsänderung ΔE ergab sich durch Subtraktion des zweiten Wertes vom ersten. Für die jeweiligen Proben ergibt sich ein Meßbereich bis zu 2 mM Ammoniumionen.

Ein Vergleich zwischen Probeansätzen und zugehörigen Kontrollen gab Aufschluß, ob die gemessenen Werte auf aus D, L-Tle-NH₂ freigesetzten oder auf bereits im Rohextrakt vorhandene Ammoniumionen zurückzuführen waren.

Anhand der Erstellung einer Eichgeraden mittels definierter Mengen Ammoniumchlorid konnte aus den Extinktionsänderungen die Ammoniumionen-Konzentration bestimmt werden.

### 10. OPA/IBC-Derivatisierung zur Bestimmung von D- und L-tertiär-Leucin mittels HPLC

Die Trennung und quantitative Bestimmung der Enantiomere D- und L-tertiär-Leucin erfolgte durch eine "chirale Derivatsierung" auf der Basis von o-Phthaldialdehyd (OPA)/N-Isobutyryl-L-Cystein bzw N-Isobutyryl-D-Cystein (Brückner, H.; Wittner R. und Godel H. (1991), Fully automated high-performance liquid Chromatographic separation of DL-amino acids derivatized with o-Phthaldialdehyde together with N-isopropyl-cysteine. Application to food samples. Anal. Biochem. 144, 204-206). Die gebildeten diastereomeren Isoindolderivaten wurden an einer RP-18-(Reversed Phase) Säule getrennt und durch Fluoreszenz detektiert. Als mobile Phase wurde ein Zwei-Puffer-System aus wäßrigem Acetat-Puffer und einem Acetonitril/Wasser-Gemisch verwendet.

### Chromatographiebedingungen:

Stationäre Phase: Kromasil^{™} HPLC-Säule, 250 × 4 mm, 5 µm, 100 Å (Firma Eka Nobel)

### Mobile Phase:

laufmittel A: 23 mM Natriumacetat, pH 6,0
Laufmittel B: Acetonitril (HPLC-Grade) und Aq. demin.: 10:1,5 (v/v)
Flußrate: 1 ml/min
Probenvolumen: 20 µl
Detektion: Fluoreszenz: Ex. 340 nm/Em. 440 nm

**Tabelle 22 Gradientenprogramm der HPLC**

| Gradientenprogramm: | |
|---|---|
| Zeit [min] | Laufmittel B [%] |
| 0 | 23 |
| 25 | 28 |
| 27 | 100 |
| 30 | 100 |
| 32 | 0 |
| 42 | 0 |

### 11. Aufreinigung der D-Amidase aus Variovorax paradoxus

Die Aufreinigung der D-Amidase erfolgte nach Zellaufschluß in drei Chromatographieschritten.
1. Ionenaustauschchromatographie:
   Säulenmaterial: Q-Sepharose - FF (Pharmacia)
   Puffer A: Kaliumphosphat-Puffer, 20 mM, pH 6, 5
   Puffer B: Puffer A + 150 mM Na2SO4
   Elution über einen linearen Gradienten
2. Hydrophobe Interaktionschromatographie :
   Säulenmaterial: Butyl-Sepharose 4 FF (Pharmacia)
   Puffer A: Kaliumphosphat-Puffer, 20 mM, pH 6,5 + 150 mM Na2SO4
   Puffer B: Kaliumphosphat-Puffer, 20 mM, pH 6,5
   Elution über einen linearen Gradienten
3. Gelfiltrationschromatographie:
   Säulenmaterial: Superdex 200 PG (Pharmacia)
   Puffer: Kaliumphosphat-Puffer, 20 mM, pH 6,5 + 180 mM NaCl

Nach dem 2. Schritt liegt meist nur noch ein sehr geringer Anteil an Fremdprotein vor. Für die ersten zwei Aufreinigungsschritte läßt sich eine Ausbeute von 88 % berechnen, wobei eine spez. Akt. von 0,68 U/mg für DL-Tle-NH₂ vorliegt. Für die homogen aufgereinigte D-Amidase aus Variovorax paradoxus ergab sich eine spez. Akt von 1,4 U/mg für DL-Tle-NH₂.

### 12. Bestimmung das Temperatur-Optimums der D-amidase

Zur Bestimmung des Temperaturoptimums diente nahezu homogen aufgereinigtes Enzym mit einer spez. Akt. von etwa 1,3 U/mg. Es erfolgte eine Bestimmung der Aktivität, wie unter 8. beschrieben, mit DL-Tle-NH₂ als Substrat von 20° bis 50°C in 5°C Schritten, wobei der Bereich von 409 bis 55°C mit weiteren Messungen näher untersucht wurde. Die Inkubationszeit wurde mit 15 min relativ kurz gewählt. Der Verlauf der Aktivität in Abhängigkeit von der Temperatur ist in Fig. 2 dargestellt.

### 13. Bestimmung des pH-Optimums, der D-Amidase

Hierzu wurde mit angereinigtem Enzym nach der Ionenaustauschchromatographie die Aktivität bei pH-Werten im Beireich von 3,5 bis 11 mit folgenden Puffersubstanzen bestimmt.

**Tabelle 23: Puffer zur Bestimmung des pH-Optimums**

| pH-Bereich | Puffersubstanz [100 mM]. |
|---|---|
| 3,5-6,0 | Na-Citrat |
| 4,0-5,0 | Na-Acetat |
| 5,0-6,5 | MES |
| 5,5-8,5 | Kpi |
| 8,0-9,5 | TRIS |
| 8,5-11,0 | Na-Carbonat |

Die Bestimmung der Aktivität erfolgte, wie unter 8. beschrieben, mit DL-Tle-NH₂ als Substrat und einer Inkubation von 30 min bei 30°C. Der Verlauf der Aktivität in Abhängigkeit vom pH-Wert und der jeweiligen Puffer-substanz ist in Fig. 3 dargestellt.

### SEQUENZPROTOKOLL

<110> Degussa AG
<120> Amidase aus Variovorax paradoxus
<130> 010199 AM
<140>
   <141>
<160> 13
<170> PatentIn Ver. 2.1
<210> 1
   <211> 1398
   <212> DNA
   <213> Variovorax paradoxus
<220>
   <221> CDS
   <222> (1)..(1398)
<400> 1
   atg agc aac gaa ctg cat tac ctc gag ctg gtc gac gtc ggc cgg cgc     48
   Met Ser Asn Glu Leu His Tyr Leu Glu Leu Val Asp Val Gly Arg Arg
     1               5                  10                  15
   atc cag cgc aag gag ctc tcg ccg gtc gag gtc acg caa gcg cag ctc     96
   Ile Gln Arg Lys Glu Leu Ser Pro Val Glu Val Thr Gln Ala Gln Leu
                20                  25                  30
   gcg cgc atc gag aag gtc gac ggc gcg ctc aag agc tat gtg atc gtg    144
   Ala Arg Ile Glu Lys Val Asp Gly Ala Leu Lys Ser Tyr Val Ile Val
            35                  40                  45
   atg gcc gaa cac gcg ctg gcc gac gcg cgc cgc gcc gag gcc gag atc    192
   Met Ala Glu His Ala Leu Ala Asp Ala Arg Arg Ala Glu Ala Glu Ile
        50                  55                  60
   gcc cgg ggc gag atc cgc ggg ccg ctg cac ggc gtg ccg gtg gcg gtg    240
   Ala Arg Gly Glu Ile Arg Gly Pro Leu His Gly Val Pro Val Ala Val
    65                  70                  75                  80
   aag gac ctg tgc tgg aca aaa ggc gtg gcc acg gcc gcc ggc atg acg    288
   Lys Asp Leu Cys Trp Thr Lys Gly Val Ala Thr Ala Ala Gly Met Thr
                    85                  90                  95
   ctc tac cgc gac ttc gtg ccc acc gag gac ggc acg gcc gtg cgc aag    336
   Leu Tyr Arg Asp Phe Val Pro Thr Glu Asp Gly Thr Ala Val Arg Lys
               100                 105                 110
   ctg cgc gaa gcc ggt gcc gtg atc ctc ggc aag ctg cag ctc acc gag    384
   Leu Arg Glu Ala Gly Ala Val Ile Leu Gly Lys Leu Gln Leu Thr Glu
           115                 120                 125
   agc gcc tat gcc gac cat cac ccc agc gtc acg ccg ccg gtc aat ccg    432
   Ser Ala Tyr Ala Asp His His Pro Ser Val Thr Pro Pro Val Asn Pro
   130                 135                 140
   tgg aac gcg gcg cac tgg tcg ggt gcc tcg tcg agc ggc tcg ggc gtg      480
   Trp Asn Ala Ala His Trp Ser Gly Ala Ser Ser Ser Gly Ser Gly Val
   145                 150                 155                 160
   gcg acc gcg gcg ggg ctt tgc tat ggc tcg ctc ggc acc gac acg ggc      528
   Ala Thr Ala Ala Gly Leu Cys Tyr Gly Ser Leu Gly Thr Asp Thr Gly
                   165                 170                 175
   ggc tcg atc cgc ttt ccg tcc tcg gcc aac ggc ctg acc ggc ctg aag      576
   Gly Ser Ile Arg Phe Pro Ser Ser Ala Asn Gly Leu Thr Gly Leu Lys
               180                 185                 190
   ccg acc tgg ggc cgc gtg agc cgc cat ggc gcc ttc gag ctg gcc gcc      624
   Pro Thr Trp Gly Arg Val Ser Arg His Gly Ala Phe Glu Leu Ala Ala
           195                 200                 205
   acg ctc gac cac atc ggc ccg atg acg cgc agc gcg gcc gat gcg ggt      672
   Thr Leu Asp His Ile Gly Pro Met Thr Arg Ser Ala Ala Asp Ala Gly
       210                 215                 220
   gcg atg ctc ggc gcc atc gcg gga gcc gat ccg aag gac ccg acc gcg      720
   Ala Met Leu Gly Ala Ile Ala Gly Ala Asp Pro Lys Asp Pro Thr Ala
   225                 230                 235                 240
   agc ctc gcg gcc gtg ccc aac tac ctc gcg ggc atg gag cgc ggc ttg      768
   Ser Leu Ala Ala Val Pro Asn Tyr Leu Ala Gly Met Glu Arg Gly Leu
                   245                 250                 255
   cgc ggc ctg cgc gtg ggc atc gac gcg cgc tgg aac gcg gag ggc gtc      816
   Arg Gly Leu Arg Val Gly Ile Asp Ala Arg Trp Asn Ala Glu Gly Val
               260                 265                 270
   gat gcg gcc acc gcg cag gtg atg gaa ggc gcg ctc gcg gcc gtg cgc      864
   Asp Ala Ala Thr Ala Gln Val Met Glu Gly Ala Leu Ala Ala Val Arg
           275                 280                 285
   gaa ctc ggc gcc gaa gtg cgt cac gtg aca ttc ccc gac ccg gcg cag      912
   Glu Leu Gly Ala Glu Val Arg His Val Thr Phe Pro Asp Pro Ala Gln
       290                 295                 300
   gtc atc gcc gac tgg ttc ccg ctg tgc ggc atc gag gcg gcc gtg gtg      960
   Val Ile Ala Asp Trp Phe Pro Leu Cys Gly Ile Glu Ala Ala Val Val
   305                 310                 315                 320
   cac gag tcg acc tat ccc gcg cgc aag cag atg tac ggc ccg gcg ctg     1008
   His Glu Ser Thr Tyr Pro Ala Arg Lys Gln Met Tyr Gly Pro Ala Leu
                   325                 330                 335
   tcg ggc ctg ctc gag ctg ggc cgt gcg caa agc ggc atc gac tac cag     1056
   Ser Gly Leu Leu Glu Leu Gly Arg Ala Gln Ser Gly Ile Asp Tyr Gln
               340                 345                 350
   aag atc gtg ctc cgc cgc cat gcc ttc agc ggc agg gtg cgc gag atg     1104
   Lys Ile Val Leu Arg Arg His Ala Phe Ser Gly Arg Val Arg Glu Met
           355                 360                 365
   ttc gag ggc atc gac ctg ctg ctg atg ccc tcg cag ggc gtc gcc tcg     1152
   Phe Glu Gly Ile Asp Leu Leu Leu Met Pro Ser Gln Gly Val Ala Ser
       370                 375                 380
   ccc acg ctc gcg cgc atg ctg agc ttc ggc gag gac gcc gaa ctg atg    1200
   Pro Thr Leu Ala Arg Met Leu Ser Phe Gly Glu Asp Ala Glu Leu Met
   385                 390                 395                 400
   tcc gcg atg ctg cgc tac acc tgc ccg ctg gac atg agc ggc agc ccg    1248
   Ser Ala Met Leu Arg Tyr Thr Cys Pro Leu Asp Met Ser Gly Ser Pro
                   405                 410                 415
   acg atc acg ctg ccc ggc ggc ttc acc gat gcg ggc acg ccg gtc gca    1296
   Thr Ile Thr Leu Pro Gly Gly Phe Thr Asp Ala Gly Thr Pro Val Ala
               420                 425                 430
   ttc cag ttc gtc gcg cgc cac ttc gag gaa gag ctg ctg gtg cgc gcc    1344
   Phe Gln Phe Val Ala Arg His Phe Glu Glu Glu Leu Leu Val Arg Ala
           435                 440                 445
   ggc tgg gcc ttc cag cag gcg acc gac tgg cac cgg cgg cat ccg gtg    1392
   Gly Trp Ala Phe Gln Gln Ala Thr Asp Trp His Arg Arg His Pro Val
       450                 455                 460
   ctg tag                                                            1398
   Leu
   465
<210> 2
   <211> 465
   <212> PRT
   <213> Variovorax paradoxus
<400> 2
   Met Ser Asn Glu Leu His Tyr Leu Glu Leu Val Asp Val Gly Arg Arg
    1               5                  10                  15
   Ile Gln Arg Lys Glu Leu Ser Pro Val Glu Val Thr Gln Ala Gln Leu
               20                  25                  30
   Ala Arg Ile Glu Lys Val Asp Gly Ala Leu Lys Ser Tyr Val Ile Val
           35                  40                  45
   Met Ala Glu His Ala Leu Ala Asp Ala Arg Arg Ala Glu Ala Glu Ile
       50                  55                  60
   Ala Arg Gly Glu Ile Arg Gly Pro Leu His Gly Val Pro Val Ala Val
   65                  70                  75                  80
   Lys Asp Leu Cys Trp Thr Lys Gly Val Ala Thr Ala Ala Gly Met Thr
                   85                  90                  95
   Leu Tyr Arg Asp Phe Val Pro Thr Glu Asp Gly Thr Ala Val Arg Lys
               100                 105                 110
   Leu Arg Glu Ala Gly Ala Val Ile Leu Gly Lys Leu Gln Leu Thr Glu
           115                 120                 125
   Ser Ala Tyr Ala Asp His His Pro Ser Val Thr Pro Pro Val Asn Pro
       130                 135                 140
   Trp Asn Ala Ala His Trp Ser Gly Ala Ser Ser Ser Gly Ser Gly Val
   145                 150                 155                 160
   Ala Thr Ala Ala Gly Leu Cys Tyr Gly Ser Leu Gly Thr Asp Thr Gly
                   165                 170                 175
   Gly Ser Ile Arg Phe Pro Ser Ser Ala Asn Gly Leu Thr Gly Leu Lys
               180                 185                 190
   Pro Thr Trp Gly Arg Val Ser Arg His Gly Ala Phe Glu Leu Ala Ala
   195 200 205
   Thr Leu Asp His Ile Gly Pro Met Thr Arg Ser Ala Ala Asp Ala Gly
   210 215 220
   Ala Met Leu Gly Ala Ile Ala Gly Ala Asp Pro Lys Asp Pro Thr Ala
   225 230 235 240
   Ser Leu Ala Ala Val Pro Asn Tyr Leu Ala Gly Met Glu Arg Gly Leu
   245 250 255
   Arg Gly Leu Arg Val Gly Ile Asp Ala Arg Trp Asn Ala Glu Gly Val
   260 265 270
   Asp Ala Ala Thr Ala Gln Val Met Glu Gly Ala Leu Ala Ala Val Arg
   275 280 285
   Glu Leu Gly Ala Glu Val Arg His Val Thr Phe Pro Asp Pro Ala Gln
   290 295 300
   Val Ile Ala Asp Trp Phe Pro Leu Cys Gly Ile Glu Ala Ala Val Val
   305 310 315 320
   His Glu Ser Thr Tyr Pro Ala Arg Lys Gln Met Tyr Gly Pro Ala Leu
   325 330 335
   Ser Gly Leu Leu Glu Leu Gly Arg Ala Gln Ser Gly Ile Asp Tyr Gln
   340 345 350
   Lys Ile Val Leu Arg Arg His Ala Phe Ser Gly Arg Val Arg Glu Met
   355 360 365
   Phe Glu Gly Ile Asp Leu Leu Leu Met Pro Ser Gln Gly Val Ala Ser
   370 375 380
   Pro Thr Leu Ala Arg Met Leu Ser Phe Gly Glu Asp Ala Glu Leu Met
   385 390 395 400
   Ser Ala Met Leu Arg Tyr Thr Cys Pro Leu Asp Met Ser Gly Ser Pro
   405 410 415
   Thr Ile Thr Leu Pro Gly Gly Phe Thr Asp Ala Gly Thr Pro Val Ala
   420 425 430
   Phe Gln Phe Val Ala Arg His Phe Glu Glu Glu Leu Leu Val Arg Ala
   435 440 445
   Gly Trp Ala Phe Gln Gln Ala Thr Asp Trp His Arg Arg His Pro Val
   450 455 460
   Leu
   465
<210> 3
   <211> 29
   <212> DNA
   <213> Künstliche Sequenz
<220>
   <223> Beschreibung der künstlichen Sequenz:Primer AAH-N1
<400> 3
   gtsggccgsc gsatccagca gaagga 26
<210> 4
   <211> 29
   <212> DNA
   <213> Künstliche Sequenz
<220>
   <223> Beschreibung der künstlichen Sequenz:Primer AAH-C1
<400> 4
   gggatscgga tcgagccgcc sgtstc 26
<210> 5
   <211> 509
   <212> DNA
   <213> Künstliche Sequenz
<220>
   <223> Beschreibung der künstlichen Sequenz:Gensonde
<400> 5
   gttggccggc gcatccagcg caaggagctc tcgccggtcg aggtcacgca agcgcagctc 60
   gcgcgcatcg agaaggtcga cggcgcgctc aagagctatg tgatcgtgat ggccgaacac 120
   gcgctggccg acgcgcgccg cgccgaggcc gagatcgccc ggggcgagat ccgcgggccg 180
   ctgcacggcg tgccggtggc ggtgaaggac ctgtgctgga caaaaggcgt ggccacggcc 240
   gccggcatga cgctctaccg cgacttcgtg cccaccgagg acggcacggc cgtgcgcaag 300
   ctgcgcgaag ccggtgccgt gatcctcggc aagctgcagc tcaccgagag cgcctatgcc 360
   gaccatcacc ccagcgtcac gccgccggtc aatccgtgga acgcggcgca ctggtcgggt 420
   gcctcgtcga gcggctcggg cgtggcgacc gcggcggggc tttgctatgg ctcgctcggc 480
   accgacacgg gcggctcgat ccgctttcc                                   509
<210> 6
   <211> 28
   <212> DNA
   <213> Künstliche Sequenz
<220>
   <223> Beschreibung der künstlichen Sequenz:Primer AAH-GW-F1
<400> 6
   gcgtcacgcc gccggtcaat ccgtggaa 28
<210> 7
   <211> 26
   <212> DNA
   <213> Künstliche Sequenz
<220>
   <223> Beschreibung der künstlichen Sequenz:Primer AAH-GW-F2
<400> 7
   ggcgcactgg tcgggtgcct cgtcga 26
<210> 8
   <211> 30
   <212> DNA
   <213> Künstliche Sequenz
<220>
   <223> Beschreibung der künstlichen Sequenz:Primer AAH-GW-R1
<400> 8
   cagcgcgtgt tcggccatca cgatcacata 30
<210> 9
   <211> 28
   <212> DNA
   <213> Künstliche Sequenz
<220>
   <223> Beschreibung der künstlichen Sequenz:Primer AAH-GW-R2
<400> 9
   ctcttgagcg cgccgtcgac cttctcga 28
<210> 10
   <211> 27
   <212> DNA
   <213> Künstliche Sequenz
<220>
   <223> Beschreibung der künstlichen Sequenz:Primer AAH-K-N2
<400> 10
   ctggtcatca agcgcggcca gatcggc 27
<210> 11
   <211> 26
   <212> DNA
   <213> Künstliche Sequenz
<220>
   <223> Beschreibung der künstlichen Sequenz:Primer AAH-K-C2
<400> 11
   gatcggccga cagccgattg gccagc 26
<210> 12
   <211> 32
   <212> DNA
   <213> Künstliche Sequenz
<220>
   <223> Beschreibung der künstlichen Sequenz:Primer AAH-N-EcoRI
<400> 12
   ccggaattca tgagcaacga actgcattac ct 32
<210> 13
   <211> 29
   <212> DNA
   <213> Künstliche Sequenz
<220>
   <223> Beschreibung der künstlichen Sequenz:Primer AAH-C-HindIII
<400> 13
   atcccaagct tttacagcac cggatgccg 29

## Patentansprüche

1. D-Amidase,
**dadurch gekennzeichnet, daß**
diese aus V. paradoxus 19-3 DSM 14468 stammt.

2. Nukleinsäuren codierend für eine Amidase gemäß Anspruch 1.

3. Plasmide, Vektoren, Mikroorganismen aufweisend eine oder mehrere klonierte Nukleinsäuren nach Anspruch 2.

4. Primer zur Herstellung der Nukleinsäuren nach Anspruch 2 mittels PCR ausgewählt aus der Gruppe bestehend aus: Seq. ID No: 3, Seq. ID No: 4, Seq. ID No: 6, Seq. ID No: 7, Seq. ID No: 8, Seq. ID No: 9, Seq. ID No: 10; Seq. ID No: 11, Seq. ID No: 12, Seq. ID No: 13.

5. Verwendung der D-Amidasen gemäß Anspruch 1 zur Herstellung von Carbonsäuren oder chiralen enantiomerenangereicherten organischen Verbindungen, wie z.B. Aminosäuren.

6. Verwendung der Nukleinsäuren gemäß Anspruch 3 zur Herstellung von Ganzzellkatalysatoren.

7. Ganzzellkatalysatoren aufweisend ein kloniertes Gen für eine Nitrilhydratase und ein kloniertes Gen für eine Amidase gemäß Anspruch 1 und optional ein kloniertes Gen für eine α-Aminonitril-Racemase, eine Cyanhydrin-Racemase, eine α-Hydroxycarbonsäure-Racemase oder eine (α- bzw. β-)Aminosäureamid-Racemase.

8. Variovorax paradoxus 19-3 DSM 14468.

## Claims

1. D-Amidase **characterized in that** it originates from V. paradoxus 19-3 DSM 14468.

2. Nucleic acids coding for an amidase according to Claim 1.

3. Plasmids, vectors, microorganisms including one or more cloned nucleic acids according to Claim 2.

4. Primers for preparing the nucleic acids according to Claim 2 by means of PCR selected from the group consisting of: Seq. ID No: 3, Seq. ID No: 4, Seq. ID No: 6, Seq. ID No: 7, Seq. ID No: 8, Seq. ID No: 9, Seq. ID No: 10, Seq. ID No: 11, Seq. ID No: 12, Seq. ID No: 13.

5. Use of the D-amidases according to Claim 1 for preparing carboxylic acids or chiral enantiomer-enriched organic compounds such as, for example, amino acids.

6. Use of the nucleic acids according to Claim 3 for preparing whole-cell catalysts.

7. Whole-cell catalysts including a cloned gene for a nitrile hydratase and a cloned gene for an amidase according to Claim 1 and optionally a cloned gene for an α-amino-nitrile racemase, a cyanohydrin racemase, an α-hydroxy-carboxylate racemase or an (α- or β-) amino-amide racemase.

8. Variovorax paradoxus 19-3 DSM 14468.

## Revendications

1. D-Amidase **caractérisée en ce qu'**elle provient de V.paradoxus 19-3 DSM 14468.

2. Acides nucléiques codant pour une amidase selon la revendication 1.

3. Plasmides, vecteurs, micro-organismes comprenant un ou plusieurs acides nucléiques clonés selon la revendication 2.

4. Amorces pour la préparation des acides nucléiques selon la revendication 2, par PCR, choisies dans le groupe constitué de :
seq. ID no.: 3, seq. ID no.: 4, seq. ID no.: 6, seq. ID no.: 7, seq. ID no.: 8, seq. ID no.: 9, seq. ID no.: 10, seq. ID no.: 11, seq. ID no.: 12, seq. ID no.: 13.

5. Utilisation des D-amidases selon la revendication 1 pour la préparation d'acides carboxyliques ou de composés organiques chiraux enrichis en énantiomères, comme par exemple des acides aminés.

6. Utilisation des acides nucléiques selon la revendication 3 pour la préparation de catalyseurs à cellules entières.

7. Catalyseurs à cellules entières comprenant un gène cloné codant par une nitrile hydratase et un gène cloné codant pour une amidase selon la revendication 1 et éventuellement un gène cloné codant pour une α-amino-nitrile racémase, une cyanohydrine racémase, une α-hydroxy-carboxylate racémase ou une α- ou β-amino-amide racémase.

8. Variovorax paradoxus 19-3 DSM 14468.
